# EUROPEAN PATENT SPECIFICATION

(11) **EP 1 414 952 B1**
(45) Date of publication and mention of the grant of the patent: **19.12.2007**
(21) Application number: 02740596.8
(22) Date of filing: 14.05.2002
(51) Int. Cl.: C12N 9/10

(54) **PROCESS FOR THE FERMENTATIVE PREPARATION OF L-AMINO ACIDS USING CORYNEFORM BACTERIA**
VERFAHREN ZUR FERMENTATIVEN HERSTELLUNG VON L-AMINOSÄUREN MITTELS KORYNEFORMEN BAKTERIEN
PROCEDE DE PREPARATION FERMENTATIVE D'ACIDES AMINES L A L'AIDE DE BACTERIES CORYNEFORMES

(30) Priority: 09.08.2001 DE 10139062
(43) Date of publication of application: 06.05.2004
(73) Proprietor: Evonik Degussa GmbH, 40474 Düsseldorf (DE)
(72) Inventor: WOLF, Andreas, 50933 Köln (DE); SCHISCHKA, Natalie, 33649 Bielefeld (DE); HERMANN, Thomas, 33739 Bielefeld (DE); MORBACH, Susanne, 52428 Jülich (DE); KRÄMER, Reinhard, 52428 Jülich (DE)
(86) International application number: PCT/EP2002/005264
(87) International publication number: WO 2003/014370

(56) References cited:
- EP-A- 1 174 508
- WO-A-97/38111
- DATABASE GENBANK [Online] Trehalose phosphatase , 24 January 2001 (2001-01-24) POMPEJUS, M. ET AL. : "Corynebacterium glutamicum genes encoding metabolic pathway proteins" retrieved from NCBI Database accession no. AX064857 XP002231768 & WO 01 00843 A (BASF AKTIENGESELLSCHAFT (DE)) 4 January 2001 (2001-01-04)
- DATABASE GENBANK [Online] Maltooligosyl-trehalose synthase, 24 January 2001 (2001-01-24) POMPEJUS, M. ET AL.: "Corynebacterium glutamicum genes encoding metabolic pathway proteins" retrieved from NCBI Database accession no. Ax064861 XP002231769 & WO 01 00843 A (BASF AKTIENGESSELLSCHAFT (DE)) 4 January 2001 (2001-01-04)
- DATABASE GENBANK [Online] Maltooligosyl-trehalose trehalohydrolase, 24 January 2001 (2001-01-24) POMPEJUS, M. ET AL.: "Corynebacterium glutamicum genes encoding metabolic pathway proteins" retrieved from NCBI Database accession no. Ax064863 XP002231770 & WO 01 00843 A (BASF AKTIENGESELLSCHAFT (DE)) 4 January 2001 (2001-01-04)
- MARTIN J F ET AL: "CLONING SYSTEMS IN AMINO ACID-PRODUCING CORYNEBACTERIA" BIO/TECHNOLOGY, NATURE PUBLISHING CO. NEW YORK, US, vol. 5, 1 February 1987 (1987-02-01), pages 137-146, XP002034056 ISSN: 0733-222X

## Description

The invention relates to a process for the fermentative preparation L-lysine using coryneform bacteria in which one or more genes chosen from the group consisting of the otsB gene, treY gene and treZ gene are attenuated.

### Prior art

L-Amino acids, in particular L-lysine and L-glutamic acid, are used in human medicine and in the pharmaceuticals industry, in the foodstuffs industry and very particularly in animal nutrition.

It is known that amino acids are prepared by fermentation from strains of coryneform bacteria, in particular Corynebacterium glutamicum. Because of their great importance, work is constantly being undertaken to improve the preparation processes. Improvements to the process can relate to fermentation measures, such as, for example, stirring and supply of oxygen, or the composition of the nutrient media, such as, for example, the sugar concentration during the fermentation, or the working up to the product form by, for example, ion exchange chromatography, or the intrinsic output properties of the microorganism itself.

Methods of mutagenesis, selection and mutant selection are used to improve the output properties of these microorganisms. Strains which are resistant to antimetabolites, such as, for example, the lysine analogue S-(2-aminoethyl)-cysteine, or are auxotrophic for metabolites of regulatory importance and produce L- amino acids are obtained in this manner.

Methods of the recombinant DNA technique have also been employed for some years for improving the strain of Corynebacterium glutamicum strains which produce L-amino acids, by amplifying individual amino acid biosynthesis genes and investigating the effect on the L-amino acid production.

### Object of the invention

The inventors had the object of providing new fundamentals for improved processes for the fermentative preparation of L-lysine with coryneform bacteria.

### Description of the invention

Where L-amino acids or amino acids are mentioned in the following, this means one or more amino acids, including their salts, chosen from the group consisting of L-asparagine, L-threonine, L-serine, L-glutamic acid, L-glycine, L-alanine, L-cysteine, L-valine, L-methionine, L-isoleucine, L-leucine, L-tyrosine, L-phenylalanine, L-histidine, L-lysine, L-tryptophan and L-arginine. L-Lysine and L-glutamic acid are particularly preferred.

When L-lysine or lysine are mentioned in the following, not only the bases but also the salts, such as e.g. lysine monohydrochloride or lysine sulfate, are meant by this.

When L-glutamic acid or glutamic acid are mentioned in the following, the salts, such as e.g. glutamic acid hydrochloride or glutamic acid sulfate are also meant by this.

The invention provides a process for the fermentative preparation of L-Lysine using coryneform bacteria in which at least the nucleotide sequence which codes for trehalose phosphatase and/or the nucleotide sequence which codes for maltooligosyl-trehalose synthase and/or the nucleotide sequence which codes for maltooligosyl-trehalose trehalohydrolase is or are attenuated, in particular eliminated or expressed at a low level.

This invention also provides a process for the fermentative preparation of L-amino acids, in which the following steps are carried out:
a) fermentation of the L-Lysine producing coryneform bacteria in which at least the nucleotide sequence which codes for trehalose phosphatase and/or the nucleotide sequence which codes for maltooligosyl-trehalose synthase and/or the nucleotide sequence which codes for maltooligosyl-trehalose trehalohydrolase is or are attenuated, in particular eliminated or expressed at a low level;
b) concentration of the L-Lysine in the medium or in the cells of the bacteria;
c) isolation of the L-Lysine, constituents of the fermentation broth and/or the biomass optionally remaining in portions or in their total amounts in the end product.

The strains employed preferably already produce L-amino acids, in particular L-lysine and L-glutamic acid, before the attenuation of the otsB gene, which codes for trehalose phosphatase, and/or the treY gene, which codes for maltooligosyl-trehalose synthase, and/or the treZ gene, which codes for maltooligosyl-trehalose trehalohydrolase.

The term "attenuation" in this connection describes the reduction or elimination of the intracellular activity of one or more enzymes (proteins) in a microorganism which are coded by the corresponding DNA, for example by using a weak promoter or using a gene or allele which codes for a corresponding enzyme with a low activity or inactivates the corresponding gene or enzyme (protein), and optionally combining these measures.

By attenuation measures, the activity or concentration of the corresponding protein is in general reduced to 0 to 75%, 0 to 50%, 0 to 25%, 0 to 10% or 0 to 5% of the activity or concentration of the wild-type protein or of the activity or concentration of the protein in the starting microorganism.

The microorganisms provided by the present invention can prepare amino acids from glucose, sucrose, lactose, fructose, maltose, molasses, starch cellulose or from glycerol and ethanol. They are representatives of coryneform bacteria, in particular of the genus Corynebacterium. Of the genus Corynebacterium, there may be mentioned in particular the species Corynebacterium glutamicum, which is known among experts for its ability to produce L-amino acids.

Suitable strains of the genus Corynebacterium, in particular of the species Corynebacterium glutamicum, are in particular the known wild-type strains
Corynebacterium glutamicum ATCC13032
Corynebacterium acetoglutamicum ATCC15806
Corynebacterium acetoacidophilum ATCC13870
Corynebacterium melassecola ATCC17965
Corynebacterium thermoaminogenes FERM BP-1539
Brevibacterium flavum ATCC14067
Brevibacterium lactofermentum ATCC13869 and
Brevibacterium divaricatum ATCC14020
and L-amino acid-producing mutants or strains prepared therefrom
such as, for example, the L-lysine-producing strains
Corynebacterium glutamicum FERM-P 1709
Brevibacterium flavum FERM-P 1708
Brevibacterium lactofermentum FERM-P 1712
Corynebacterium glutamicum FERM-P 6463
Corynebacterium glutamicum FERM-P 6464 and
Corynebacterium glutamicum DSM 5715.

It has been found that coryneform bacteria produce L-Lysine acids in an improved manner after attenuation of the otsB gene, which codes for trehalose phosphatase (EC: 3.1.3.12), and/or the treY gene, which codes for maltooligosyl-trehalose synthase, and/or the treZ gene, which codes for maltooligosyl-trehalose trehalohydrolase.

The nucleotide sequence of the gene which codes for the trehalose phosphatase of Corynebacterium glutamicum can be found in the patent application WO 01/00843 under Identification Code RXA00347 as SEQ ID No. 1139.

The nucleotide sequence of the gene which codes for the maltooligosyl-trehalose synthase of Corynebacterium glutamicum can be found in the patent application WO 01/00843 under Identification Code FRXA01239 as SEQ ID No. 1143.

The nucleotide sequence of the gene which codes for the maltooligosyl-trehalose trehalohydrolase of Corynebacterium glutamicum can be found in the patent application WO 01/00843 under Identification Code RXA02645 as SEQ ID No. 1145.

The nucleotide sequences are also deposited in the gene library under Accession Number AX064857, AX064861 and AX064863.

The nucleotide sequences claimed, of the genes which code for trehalose phosphatase, for maltooligosyl-trehalose synthase and for maltooligosyl-trehalose trehalohydrolase, shown in SEQ ID No. 1, SEQ ID No. 3 or SEQ ID No. 5 are lengthened compared with the sequences known from the prior art by in each case preferably up to 700 base pairs before the start codon and after the stop codon of the gene.

The lengthenings compared with the sequence known from the prior art comprise base pairs 1 to 500 and 1392 to 1977 in SEQ ID No. 1.

In SEQ ID No. 3 the lengthenings compared with the sequence known from the prior art comprise base pairs 1 to 500 and 3057 to 3636.

In SEQ ID No. 5 the lengthenings compared with the sequence known from the prior art comprise base pairs 1 to 500 and 2454 to 3033.

The amino acid sequences of the associated gene products are shown in SEQ ID No. 2, SEQ ID No. 4 or SEQ ID No. 6.

It has been found that attenuation processes which are known per se can be employed particularly successfully with the aid of the lengthened sequences thus provided.

Such a process is the method of gene replacement. In this, a mutation, such as e.g. a deletion, insertion or base exchange, is established in vitro in the gene of interest. The allele prepared is in turn cloned in a vector which is not replicative for C. glutamicum and this is then transferred into the desired host of C. glutamicum by transformation or conjugation. After homologous recombination by means of a first "cross-over" event which effects integration and a suitable second "cross-over" event which effects excision in the target gene or in the target sequence, the incorporation of the mutation or of the allele is achieved. This method was used, for example, in EP: 00110021.3 to eliminate the secG gene of C. glutamicum.

The lengthening of the sequences employed is not limited to 600 base pairs before the start codon and after the stop codon. It is preferably in the range from 300 to 700 base pairs, but can also be up to 800 base pairs. The lengthenings can also contain different amounts of base pairs.

The sequences described in the text references mentioned which code for trehalose phosphatase, maltooligosyl-trehalose synthase and maltooligosyl-trehalose trehalohydrolase can be used according to the invention. Alleles of trehalose phosphatase, maltooligosyl-trehalose synthase or maltooligosyl-trehalose trehalohydrolase which result from the degeneracy of the genetic code or due to "sense mutations" of neutral function can furthermore be used.

To achieve an attenuation, either the expression of the gene which codes for trehalose phosphatase and/or the expression of the gene which codes for maltooligosyl-trehalose synthase and/or the expression of the gene which codes for maltooligosyl-trehalose trehalohydrolase or the catalytic properties of the gene products can be reduced or eliminated. The two measures are optionally combined.

The gene expression can be reduced by suitable culturing or by genetic modification (mutation) of the signal structures of gene expression. Signal structures of gene expression are, for example, repressor genes, activator genes, operators, promoters, attenuators, ribosome binding sites, the start codon and terminators. The expert can find information on this e.g. in the patent application WO 96/15246, in Boyd and Murphy (Journal of Bacteriology 170: 5949 (1988)), in Voskuil and Chambliss (Nucleic Acids Research 26: 3548 (1998), in Jensen and Hammer (Biotechnology and Bioengineering 58: 191 (1998)), in Pátek et al. (Microbiology 142: 1297 (1996)) and in known textbooks of genetics and molecular biology, such as e.g. the textbook by Knippers ("Molekulare Genetik", 6th edition, Georg Thieme Verlag, Stuttgart, Germany, 1995) or that by Winnacker ("Gene und Klone", VCH Verlagsgesellschaft, Weinheim, Germany, 1990).

Mutations which lead to a change or reduction in the catalytic properties of enzyme proteins are known from the prior art; examples which may be mentioned are the works by Qiu and Goodman (Journal of Biological Chemistry 272: 8611-8617 (1997)), Sugimoto et al. (Bioscience Biotechnology and Biochemistry 61: 1760-1762 (1997)) and Möckel ("Die Threonindehydratase aus Corynebacterium glutamicum: Aufhebung der allosterischen Regulation und Struktur des Enzyms", Reports from the Jülich Research Centre, Jül-2906, ISSN09442952, Jülich, Germany, 1994). Summarizing descriptions can be found in known textbooks of genetics and molecular biology, such as e.g. that by Hagemann ("Allgemeine Genetik", Gustav Fischer Verlag, Stuttgart, 1986).

Possible mutations are transitions, transversions, insertions and deletions. Depending on the effect of the amino acid exchange on the enzyme activity, "missense mutations" or "nonsense mutations" are referred to. Insertions or deletions of at least one base pair in a gene lead to "frame shift mutations", as a consequence of which incorrect amino acids are incorporated or translation is interrupted prematurely. Deletions of several codons typically lead to a complete loss of the enzyme activity. Instructions on generation of such mutations are prior art and can be found in known textbooks of genetics and molecular biology, such as e.g. the textbook by Knippers ("Molekulare Genetik", 6th edition, Georg Thieme Verlag, Stuttgart, Germany, 1995), that by Winnacker ("Gene und Klone", VCH Verlagsgesellschaft, Weinheim, Germany, 1990) or that by Hagemann ("Allgemeine Genetik", Gustav Fischer Verlag, Stuttgart, 1986).

A common method of mutating genes of C. glutamicum is the method of "gene disruption" and "gene replacement" described by Schwarzer and Pühler (Bio/Technology 9, 84-87 (1991)).

In the method of gene disruption a central part of the coding region of the gene of interest is cloned in a plasmid vector which can replicate in a host (typically E. coli), but not in C. glutamicum. Possible vectors are, for example, pSUP301 (Simon et al., Bio/Technology 1, 784-791 (1983)), pK18mob or pK19mob (Schäfer et al., Gene 145, 69-73 (1994)), pK18mobsacB or pK19mobsacB (Jäger et al., Journal of Bacteriology 174: 5462-65 (1992)), pGEM-T (Promega Corporation, Madison, WI, USA), pCR2.1-TOPO (Shuman (1994). Journal of Biological Chemistry 269:32678-84; US Patent 5,487,993), pCR®Blunt (Invitrogen, Groningen, Holland; Bernard et al., Journal of Molecular Biology, 234: 534-541 (1993)) or pEM1 (Schrumpf et al, 1991, Journal of Bacteriology 173:4510-4516). The plasmid vector which contains the central part of the coding region of the gene is then transferred into the desired strain of C. glutamicum by conjugation or transformation. The method of conjugation is described, for example, by Schäfer et al. (Applied and Environmental Microbiology 60, 756-759 (1994)). Methods for transformation are described, for example, by Thierbach et al. (Applied Microbiology and Biotechnology 29, 356-362 (1988)), Dunican and Shivnan (Bio/Technology 7, 1067-1070 (1989)) and Tauch et al. (FEMS Microbiological Letters 123, 343-347 (1994)). After homologous recombination by means of a "cross-over" event, the coding region of the gene in question is interrupted by the vector sequence and two incomplete alleles are obtained, one lacking the 3' end and one lacking the 5' end. This method has been used, for example, by Fitzpatrick et al. (Applied Microbiology and Biotechnology 42, 575-580 (1994)) to eliminate the recA gene of C. glutamicum.

In the method of "gene replacement", a mutation, such as e.g. a deletion, insertion or base exchange, is established in vitro in the gene of interest. The allele prepared is in turn cloned in a vector which is not replicative for C. glutamicum and this is then transferred into the desired host of C. glutamicum by transformation or conjugation. After homologous recombination by means of a first "cross-over" event which effects integration and a suitable second "cross-over" event which effects excision in the target gene or in the target sequence, the incorporation of the mutation or of the allele is achieved. This method was used, for example, by Peters-Wendisch et al. (Microbiology 144, 915 - 927 (1998)) to eliminate the pyc gene of C. glutamicum by a deletion.

A deletion, insertion or a base exchange can be incorporated in this manner into the gene which codes for trehalose phosphatase and/or the gene which codes for maltooligosyl-trehalose synthase and/or the gene which codes for maltooligosyl-trehalose trehalohydrolase.

In addition, it may be advantageous for the production of L-amino acids to enhance, in particular over-express, one or more enzymes of the particular biosynthesis pathway, of glycolysis, of anaplerosis, of the citric acid cycle, of the pentose phosphate cycle, of amino acid export and optionally regulatory proteins, in addition to the attenuation of the gene which codes for trehalose phosphatase and/or the gene which codes for maltooligosyl-trehalose synthase and/or the gene which codes for maltooligosyl-trehalose trehalohydrolase.

The term "enhancement" or "enhance" in this connection describes the increase in the intracellular activity of one or more enzymes or proteins in a microorganism which are coded by the corresponding DNA, for example by increasing the number of copies of the gene or genes, using a potent promoter or a gene which codes for a corresponding enzyme or protein with a high activity, and optionally combining these measures.

By enhancement measures, in particular over-expression, the activity or concentration of the corresponding protein is in general increased by at least 10%, 25%, 50%, 75%, 100%, 150%, 200%, 300%, 400% or 500%, up to a maximum of 1000% or 2000%, based on that of the wild-type protein or the activity or concentration of the protein in the starting microorganism.

Thus, for the production of L-lysine in addition to the attenuation of the gene which codes for trehalose phosphatase and/or the gene which codes for maltooligosyl-trehalose synthase and/or the gene which codes for maltooligosyl-trehalose trehalohydrolase, one or more of the genes chosen from the group consisting of
- the lysC gene which codes for a feed-back resistant aspartate kinase (Accession No.P26512, EP-B-0387527; EP-A-0699759; WO 00/63388),
- the dapA gene which codes for dihydrodipicolinate synthase (EP-B 0 197 335),
- the gap gene which codes for glyceraldehyde 3-phosphate dehydrogenase (Eikmanns (1992). Journal of Bacteriology 174:6076-6086),
- at the same time the pyc gene which codes for pyruvate carboxylase (DE-A-198 31 609),
- the mqo gene which codes for malate:quinone oxidoreductase (Molenaar et al., European Journal of Biochemistry 254, 395-403 (1998)),
- the zwf gene which codes for glucose 6-phosphate dehydrogenase (JP-A-09224661),
- at the same time the lysE gene which codes for lysine export (DE-A-195 48 222),
- the zwa1 gene which codes for the Zwa1 protein (DE: 19959328.0, DSM 13115)
- the tpi gene which codes for triose phosphate isomerase (Eikmanns (1992), Journal of Bacteriology 174:6076-6086), and
- the pgk gene which codes for 3-phosphoglycerate kinase (Eikmanns (1992), Journal of Bacteriology 174:6076-6086),
can be enhanced, in particular over-expressed.

It may furthermore be advantageous for the production of L-Lysine, in addition to the attenuation of the gene which codes for trehalose phosphatase and/or the gene which codes for maltooligosyl-trehalose synthase and/or the gene which codes for maltooligosyl-trehalose trehalohydrolase, at the same time for one or more of the genes chosen from the group consisting of
- the pck gene which codes for phosphoenol pyruvate carboxykinase (DE 199 50 409.1, DSM 13047),
- the pgi gene which codes for glucose 6-phosphate isomerase (US 09/396,478, DSM 12969),
- the poxB gene which codes for pyruvate oxidase (DE:1995 1975.7, DSM 13114),
- the zwa2 gene which codes for the Zwa2 protein (DE: 19959327.2, DSM 13113),
- the hom gene which codes for homoserine dehydrogenase (EP-A -0131171) and
- the thrB gene which codes for homoserine kinase (Peoples, O.W., et al., Molecular Microbiology 2 (1988): 63 - 72)
to be attenuated, in particular for the expression thereof to be reduced.

Finally, it may be advantageous for the production of Lysine, in addition to the attenuation of the gene which codes for trehalose phosphatase and/or the gene which codes for maltooligosyl-trehalose synthase and/or the gene which codes for maltooligosyl-trehalose trehalohydrolase, to eliminate undesirable side reactions (Nakayama: "Breeding of Amino Acid Producing Micro-organisms", in: Overproduction of Microbial Products, Krumphanzl, Sikyta, Vanek (eds.), Academic Press, London, UK, 1982).

The invention also provides the microorganisms prepared according to the invention, and these can be cultured continuously or discontinuously in the batch process (batch culture) or in the fed batch (feed process) or repeated fed batch process (repetitive feed process) for the purpose of production of L-amino acids. A summary of known culture methods is described in the textbook by Chmiel (Bioprozesstechnik 1. Einführung in die Bioverfahrenstechnik (Gustav Fischer Verlag, Stuttgart, 1991)) or in the textbook by Storhas (Bioreaktoren und periphere Einrichtungen (Vieweg Verlag, Braunschweig/Wiesbaden, 1994)).

The culture medium to be used must meet the requirements of the particular strains in a suitable manner. Descriptions of culture media for various microorganisms are contained in the handbook "Manual of Methods for General Bacteriology" of the American Society for Bacteriology (Washington D.C., USA, 1981).

Sugars and carbohydrates, such as e.g. glucose, sucrose, lactose, fructose, maltose, molasses, starch and cellulose, oils and fats, such as e.g. soya oil, sunflower oil, groundnut oil and coconut fat, fatty acids, such as e.g. palmitic acid, stearic acid and linoleic acid, alcohols, such as e.g. glycerol and ethanol, and organic acids, such as e.g. acetic acid, can be used as the source of carbon. These substances can be used individually or as a mixture.

Organic nitrogen-containing compounds, such as peptones, yeast extract, meat extract, malt extract, corn steep liquor, soya bean flour and urea, or inorganic compounds, such as ammonium sulfate, ammonium chloride, ammonium phosphate, ammonium carbonate and ammonium nitrate, can be used as the source of nitrogen. The sources of nitrogen can be used individually or as a mixture.

Phosphoric acid, potassium dihydrogen phosphate or dipotassium hydrogen phosphate or the corresponding sodium-containing salts can be used as the source of phosphorus. The culture medium must furthermore comprise salts of metals, such as e. g. magnesium sulfate or iron sulfate, which are necessary for growth. Finally, essential growth substances, such as amino acids and vitamins, can be employed in addition to the abovementioned substances. Suitable precursors can moreover be added to the culture medium. The starting substances mentioned can be added to the culture in the form of a single batch, or can be fed in during the culture in a suitable manner.

Basic compounds, such as sodium hydroxide, potassium hydroxide, ammonia or aqueous ammonia, or acid compounds, such as phosphoric acid or sulfuric acid, can be employed in a suitable manner to control the pH of the culture. Antifoams, such as e.g. fatty acid polyglycol esters, can be employed to control the development of foam. Suitable substances having a selective action, such as e.g. antibiotics, can be added to the medium to maintain the stability of plasmids. To maintain aerobic conditions, oxygen or oxygen-containing gas mixtures, such as e.g. air, are introduced into the culture. The temperature of the culture is usually 20ºC to 45ºC, and preferably 25ºC to 40ºC. Culturing is continued until a maximum of the desired product has formed. This target is usually reached within 10 hours to 160 hours.

Methods for the determination of L-amino acids are known from the prior art. The analysis can thus be carried out as described by Spackman et al. (Analytical Chemistry, 30, (1958), 1190) by anion exchange chromatography with subsequent ninhydrin derivatization, or it can be carried out by reversed phase HPLC, for example as described by Lindroth et al. (Analytical Chemistry (1979) 51: 1167-1174).

The present invention is explained in more detail in the following with the aid of embodiment examples.

### Example 1

### Preparation of integration vectors for integration mutagenesis of the otsB, treY and treZ genes

From the strain ATCC 13032, chromosomal DNA is isolated by the method of Eikmanns et al. (Microbiology 140: 1817-1828 (1994) ) .

On the basis of the sequence of the otsB, treY and treZ genes known for C. glutamicum (WO 01/00843), the following oligonucleotides are chosen for the polymerase chain reaction :
otsB-int1:
   5' GTC CGA TTT TGA TGG AAC C 3'
otsB-int2 :
   5' GGA GCT GAT GGA GTA TTC G 3'
treY-int1 :
   5' TTT TCC GTG AAT ACG TTG G 3'
treY-int2:
   5' GCG ACT AAT TCG ATG ATG G 3'
trez-intl:
   5' TGG TTC GAA GAT TTT CAC G 3'
treZ-int2:
   5' GGC GAG CTG TAG ATA ATG G 3'

The primers shown are synthesized by MWG Biotech (Ebersberg, Germany) and the PCR reaction is carried out by the standard PCR method of Innis et al. (PCR Protocols. A Guide to Methods and Applications, 1990, Academic Press) with the Taq-polymerase from Boehringer Mannheim (Germany, Product Description Taq DNA polymerase, Product No. 1 146 165). With the aid of the polymerase chain reaction, the primers allow amplification of an internal fragment of the otsB gene 463 bp in size, an internal fragment of the treY gene 530 bp in size and an internal fragment of the treZ gene 530 bp in size. The products amplified in this way are tested electrophoretically in a 0.8% agarose gel.

The amplified DNA fragment [sic] are ligated with the TOPO TA Cloning Kit from Invitrogen Corporation (Carlsbad, CA, USA; Catalogue Number K4500-01) in each case in the vector pCR2.1-TOPO (Mead at al. (1991) Bio/Technology 9:657-663).

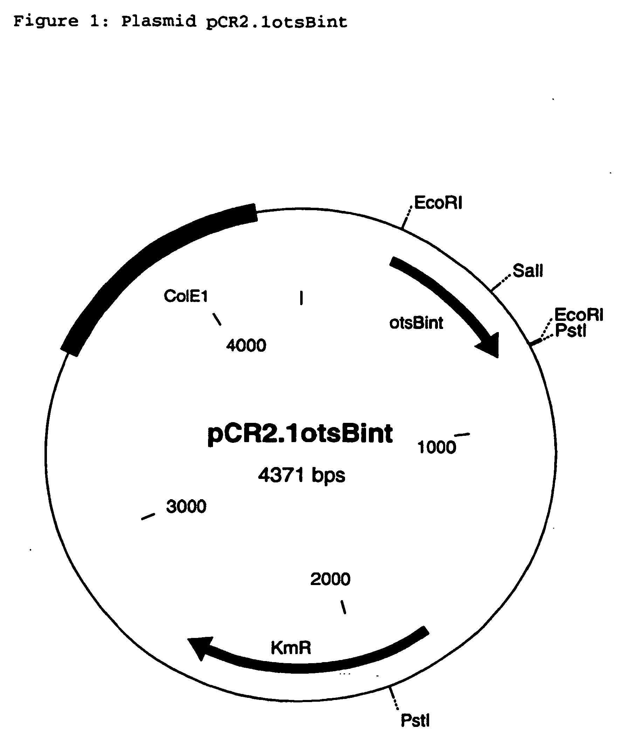

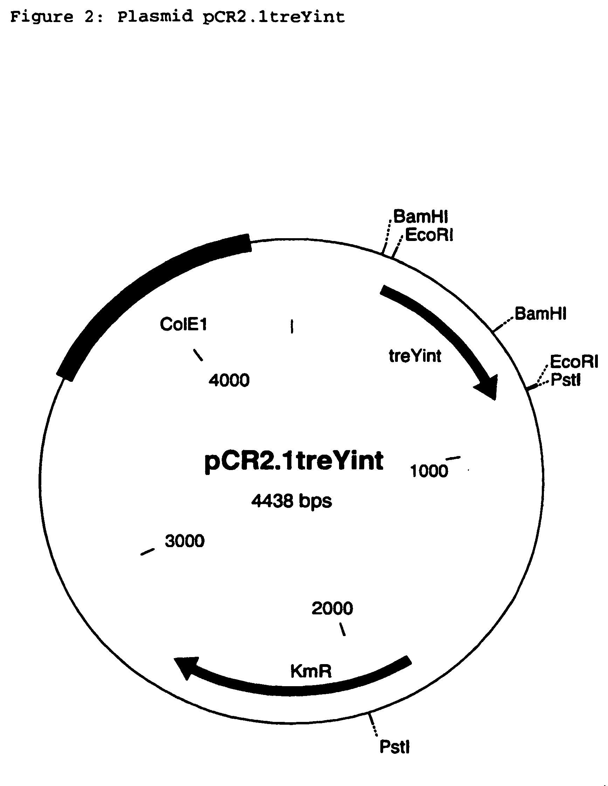

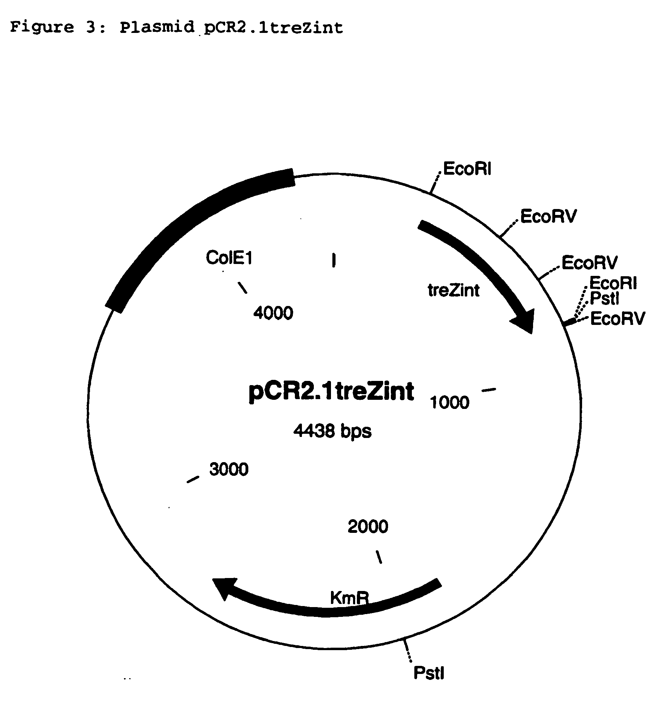

The E. coli strain TOP10 is then electroporated with the ligation batches (Hanahan, In: DNA Cloning. A Practical Approach. Vol. I, IRL-Press, Oxford, Washington DC, USA, 1985). Selection for plasmid-carrying cells is made by plating out the transformation batch on LB agar (Sambrook et al., Molecular Cloning: A Laboratory Manual. 2nd Ed., Cold Spring Harbor Laboratory Press, Cold Spring Harbor, N.Y., 1989), which had been supplemented with 50 mg/l kanamycin. Plasmid DNA is isolated from in each case one transformant with the aid of the QIAprep Spin Miniprep Kit from Qiagen and checked by restriction with the restriction enzyme EcoRI and subsequent agarose gel electrophoresis (0.8%). The plasmids are called pCR2.lotsBint, pCR2.1treYint and pCR2.1trezint and are shown in figure 1, figure 2 and figure 3.

The following microorganisms are [sic] deposited as a pure culture on 24th April 2001 at the Deutsche Sammlung für Mikroorganismen und Zellkulturen (DSMZ = German Collection of Microorganisms and Cell Cultures, Braunschweig, Germany) in accordance with the Budapest Treaty:
- Escherichia coli Top10/pCR2.1otsBint as DSM 14259,
- Escherichia coli Top10/pCR2.1treYint as DSM 14260,
- Escherichia coli Top10/pCR2.1treZint as DSM 14261.

### Example 2

### Integration mutagenesis of the otsB gene in the strain DSM 5715

The vector pCR2.1otsBint mentioned in example 1 is electroporated by the electroporation method of Tauch et al.(FEMS Microbiological Letters, 123:343-347 (1994)) in Corynebacterium glutamicum DSM 5715. The strain DSM 5715 is an AEC-resistant lysine producer. The vector pCR2.1otsBint cannot replicate independently in DSM5715 and is retained in the cell only if it has integrated into the chromosome of DSM 5715. Selection of clones with pCR2.1otsBint integrated into the chromosome is carried out by plating out the electroporation batch on LB agar (Sambrook et al., Molecular cloning: A Laboratory Manual. 2nd Ed., Cold Spring Harbor Laboratory Press, Cold Spring Harbor, N.Y.), which had been supplemented with 15 mg/l kanamycin.

For detection of the integration, the otsBint fragment is labelled with the Dig hybridization kit from Boehringer by the method of "The DIG System Users Guide for Filter Hybridization" of Boehringer Mannheim GmbH (Mannheim, Germany, 1993). Chromosomal DNA of a potential integrant is isolated by the method of Eikmanns et al. (Microbiology 140: 1817 - 1828 (1994)) and in each case cleaved with the restriction enzymes EcoRI, SalI and PstI. The fragments formed are separated by means of agarose gel electrophoresis and hybridized at 68ºC with the Dig hybridization kit from Boehringer. The plasmid pCR2.1otsBint mentioned in example 3 [sic] has been inserted into the chromosome of DSM5715 within the chromosomal otsB gene. The strain is called DSM5715::pCR2.1otsBint.

### Example 3

### Integration mutagenesis of the treY gene in the strain DSM 5715

The vector pCR2.1treYint mentioned in example 1 is electroporated by the electroporation method of Tauch et al.(FEMS Microbiological Letters, 123:343-347 (1994)) in Corynebacterium glutamicum DSM 5715. The strain DSM 5715 is an AEC-resistant lysine producer. The vector pCR2. 1treYint cannot replicate independently in DSM5715 and is retained in the cell only if it has integrated into the chromosome of DSM 5715. Selection of clones with pCR2.1treYint integrated into the chromosome is carried out by plating out the electroporation batch on LB agar (Sambrook et al., Molecular cloning: A Laboratory Manual. 2nd Ed., Cold Spring Harbor Laboratory Press, Cold Spring Harbor, N.Y.), which had been supplemented with 15 mg/l kanamycin.

For detection of the integration, the treYint fragment is labelled with the Dig hybridization kit from Boehringer by the method of "The DIG System Users Guide for Filter Hybridization" of Boehringer Mannheim GmbH (Mannheim, Germany, 1993). Chromosomal DNA of a potential integrant is isolated by the method of Eikmanns et al. (Microbiology 140: 1817 - 1828 (1994)) and in each case cleaved with the restriction enzymes EcoRI, BamHI and PstI. The fragments formed are separated by means of agarose gel electrophoresis and hybridized at 68ºC with the Dig hybridization kit from Boehringer. The plasmid pCR2.1treYint mentioned in example 3 [sic] has been inserted into the chromosome of DSM5715 within the chromosomal treY gene. The strain is called DSM5715::pCR2.1treYint.

### Example 4

### Integration mutagenesis of the treZ gene in the strain DSM 5715

The vector pCR2.1treZint mentioned in example 1 is electroporated by the electroporation method of Tauch et al.(FEMS Microbiological Letters, 123:343-347 (1994)) in Corynebacterium glutamicum DSM 5715. The strain DSM 5715 is an AEC-resistant lysine producer. The vector pCR2.1treZint cannot replicate independently in DSM5715 and is retained in the cell only if it has integrated into the chromosome of DSM 5715. Selection of clones with pCR2.1treZint integrated into the chromosome is carried out by plating out the electroporation batch on LB agar (Sambrook et al., Molecular cloning: A Laboratory Manual. 2nd Ed., Cold Spring Harbor Laboratory Press, Cold Spring Harbor, N.Y.), which had been supplmented with 15 mg/l kanamycin.

For detection of the integration, the treZint fragment is labelled with the Dig hybridization kit from Boehringer by the method of "The DIG System Users Guide for Filter Hybridization" of Boehringer Mannheim GmbH (Mannheim, Germany, 1993). Chromosomal DNA of a potential integrant is isolated by the method of Eikmanns et al. (Microbiology 140: 1817 - 1828 (1994)) and in each case cleaved with the restriction enzymes EcoRI, EcoRV and PstI. The fragments formed are separated by means of agarose gel electrophoresis and hybridized at 68°C with the Dig hybridization kit from Boehringer. The plasmid pCR2.1treZint mentioned in example 3 [sic] has been inserted into the chromosome of DSM5715 within the chromosomal treZ gene. The strain is called DSM5715::pCR2.1treZint.

### Example 5

### Preparation of lysine

The C. glutamicum strains DSM5715::pCR2.1otsBint, DSM5715::pCR2.ltreYint and DSM5715::pCR2.1treZint obtained in example 2, example 3 and example 4 are cultured in a nutrient medium suitable for the production of lysine and the lysine content in the culture supernatant is determined.

For this, the strains are first incubated on an agar plate with the corresponding antibiotic (brain-heart agar with kanamycin (25 mg/l) for 24 hours at 33°C. Starting from this agar plate culture, in each case a preculture is seeded (10 ml medium in a 100 ml conical flask). The complete medium CgIII is used as the medium for the preculture.

| Medium Cg III | |
|---|---|
| NaCl | 2.5 g/l |
| Bacto-Peptone | 10 g/l |
| Bacto-Yeast extract | 10 g/l |
| Glucose (autoclaved separately) | 2% (w/v) |

The pH is brought to pH 7.4 Kanamycin (25 mg/1) is added to this. The precultures are incubated for 16 hours at 33ºC at 240 rpm on a shaking machine. In each case a main culture is seeded from these precultures such that the initial OD (660 nm) of the main cultures is 0.1. Medium MM is used for the main culture.

| Medium MM | |
|---|---|
| CSL (corn steep liquor) | 5 g/l |
| MOPS (morpholinopropanesulfonic acid) | 20 g/l |
| Glucose (autoclaved separately) | 50g/l |
| Salts: | |
| (NH₄)₂SO₄ | 25 g/l |
| KH₂PO₄ | 0.1 g/l |
| MgSO₄ * 7 H₂O | 1.0 g/l |
| CaCl₂ * 2 H₂O | 10 mg/l |
| FeSO₄ * 7 H₂O | 10 mg/l |
| MnSO₄ * H₂O | 5.0mg/l |
| Biotin (sterile-filtered) | 0.3 mg/l |
| Thiamine * HCl (sterile-filtered) | 0.2 mg/l |
| Leucine (sterile-filtered) | 0.1 g/l |
| CaCO₃ | 25 g/l |

The CSL, MOPS and the salt solution are brought to pH 7 with aqueous ammonia and autoclaved. The sterile substrate and vitamin solutions are then added, and the CaCO₃ autoclaved in the dry state is added.

Culturing is carried out in a 10 ml volume in 100 ml conical flasks with baffles. Kanamycin (25 mg/l) was added. Culturing is carried out at 33ºC and 80% atmospheric humidity.

After 72 hours, the OD is determined at a measurement wavelength of 660 nm with a Biomek 1000 (Beckmann Instruments GmbH, Munich). The amount of lysine formed is in each case determined with an amino acid analyzer from Eppendorf-BioTronik (Hamburg, Germany) by ion exchange chromatography and post-column derivatization with ninhydrin detection.

The result of the experiment is shown in table 1.

**Table 1**

| Strain | OD (660 nm) | Lysine HCl g/l |
|---|---|---|
| DSM5715 | 7.3 | 12.48 |
| DSM5715::pCR2.1otsBint | 7.5 | 13.45 |
| DSM5715: :pCR2.1treYint | 7.5 | 13.13 |
| DSM5715::pCR2.1treZint | 8.1 | 13.84 |

### Brief description of the figures:

Figure 1: Map of the plasmid pCR2.1otsBint,
Figure 2: Map of the plasmid pCR2.1treYint,
Figure 3: Map of the plasmid pCR2.1treZint.

The abbreviations and designations used have the following meaning.
- KmR:: Kanamycin resistance gene
- BamHI:: Cleavage site of the restriction enzyme KpnI
- EcoRI:: Cleavage site of the restriction enzyme EcoRI
- EcoRV:: Cleavage site of the restriction enzyme EcoRV
- PstI:: Cleavage site of the restriction enzyme PstI
- SalI:: Cleavage site of the restriction enzyme SalI
- otsBint:: Internal fragment of the otsB gene
- treYint:: Internal fragment of the treY gene
- treZint:: Internal fragment of the treZ gene
- ColE1:: Replication origin of the plasmid ColE1

## Claims

1. A process for the fermentative preparation of L-lysine which comprises carrying out the following steps,
a) fermentation of a bacterium of the genus Corynebacterium which produce said L-lysine and in which at least one gene selected from the group consisting of a gene coding for trehalose phosphatase, a gene coding for maltooligosyl-trehalose synthase and a gene coding for maltooligosyl-trehalose trehalohydrolase is eliminated,
b) concentration of the L-lysine in the medium or in the cells of the Corynebacterium glutamicum and
c) isolation of the L-lysine optionally with constituents of the fermentation broth and/or biomass remaining in portions or in their total amounts in the end produce.

2. The process of claim 1, wherein said bacterium of the genus Corynebacterium is Corynebacterium glutamicum.

3. The process of claim 1 or 2, wherein said trehalose phosphatase comprises the amino acid sequence of SEQ ID NO: 2, wherein said maltooligosyl-trehalose synthase comprises the amino acid sequence of SEQ ID NO: 4 and wherein said maltooligosyl-trehalose trehalohydrolase comprises the amino acid sequence of SEQ ID NO: 6.

4. The process of claim 1 or 2, wherein said trehalose phosphatase gene comprises the nucleotide sequence of SEQ ID NO: 1, wherein said maltooligosyl-trehalose synthase gene comprises the nucleotide sequence of SEQ ID NO: 3 and wherein said maltooligosyl-trehalose trehalohydrolase gene comprises the nucleotide sequence of SEQ ID NO: 5.

5. The process of claim 4, wherein said trehalose phosphatase gene comprises the nucleotide sequence of nucleotides 601 to 1368 of SEQ ID NO: 1, wherein said maltooligosyl-trehalose synthase gene comprises the nucleotide sequence of nucleotides 601 to 3033 of SEQ ID NO: 3 and wherein said maltooligosyl-trehalose trehalohydrolase gene comprises the nucleotide' sequence of nucleotides 601 to 2430 of SEQ ID NO: 5.

6. The process of any of the claims 1 to 4, wherein one or more genes selected from the group consisting of
6.1 gene coding for aspartate kinase,
6.2 gene coding for dihydrodipicolinate synthase,
6.3 gene coding for glyceraldehyde 3-phosphate dehydrogenase,
6.4 gene coding for pyruvate carboxylase,
6.5 gene coding for malate:quinone oxidoreductase,
6.6 gene coding for glucose 6-phosphate dehydrogenase,
6.7 gene coding for a protein that exports lysine,
6-8 gene coding for the Zwa1 protein,
6.9 gene coding for triose phosphate isomerase, and
6.10 gene coding for 3-phosphoglycerate kinase,
is or are over-expressed.

7. The process of any of the claims 1 to 4, wherein one or more genes selected from the group consisting of
7.1 gene coding for phosphoenol pyruvate carboxykinase,
7.2 gene coding for glucose 6-phosphate isomerase,
7.3 gene coding for pyruvate oxidase,
7.4 gene coding for the Zwa2 protein,
7.5 gene coding for homoserine dehydrogenase
7.6 gene coding for homoserine kinase,
is or are eliminated.

8. The process of any of the claims 1 to 4, wherein for insertion mutagenesis of said gene coding for trehalose phosphatase plasmid pCR2.1otsBint deposited under DSM 14259 is used.

9. The process of any of the claims 1 to 4, wherein for insertion mutagenesis of said gene coding for maltooligosyl-trehalose synthase plasmid pCR2.1treYint deposited under DSM 14260is used.

10. The process of any of the claims 1 to 4, wherein for insertion mutagenesis of said gene coding for maltooligosyl-trehalose trehalohydrolase plasmid pCR2. 1trezint deposited under DSM 14261 is used.

## Patentansprüche

1. Verfahren zur fermentativen Herstellung von L-Lysin, bei dem man die folgenden Schritte durchführt:
a) Fermentation eines Bakteriums der Gattung Corynebacterium, das das L-Lysin produziert und in dem wenigstens ein aus der Gruppe, bestehend aus einem für Trehalose-Phosphatase codierenden Gen, einem für Maltooligosyl-Trehalose-Synthase codierenden Gen und einem für Maltooligosyl-Trehalose-Trehalohydrolase codierenden Gen, ausgewähltes Gen ausgeschaltet ist;
b) Anreicherung des L-Lysins im Medium oder in den Zellen des Corynebacterium glutamicum und
c) Isolierung des L-Lysins, wobei gegebenenfalls Bestandteile der Fermentationsbrühe und/oder Biomasse teilweise oder insgesamt im Endprodukt verbleiben.

2. Verfahren nach Anspruch 1, wobei es sich bei dem Bakterium der Gattung Corynebacterium um Corynebacterium glutamicum handelt.

3. Verfahren nach Anspruch 1 oder 2, wobei die Trehalose-Phosphatase die Aminosäuresequenz der SEQ ID NO: 2, die Maltooligosyl-Trehalose-Synthase die Aminosäuresequenz der SEQ ID NO: 4 und die Maltooligosyl-Trehalose-Trehalohydrolase die Aminosäuresequenz der SEQ ID NO: 6 umfaßt.

4. Verfahren nach Anspruch 1 oder 2, wobei das Trehalose-Phosphatase-Gen die Nukleotidsequenz der SEQ ID NO: 1, das Maltooligosyl-Trehalose-Synthase-Gen die Nukleotidsequenz der SEQ ID NO: 3 und das Maltooligosyl-Trehalose-Trehalohydrolase-Gen die Nukleotidsequenz der SEQ ID NO: 5 umfaßt.

5. Verfahren nach Anspruch 4, wobei das Trehalose-Phosphatase-Gen die Nukleotidsequenz der Nukleotide 601 bis 1368 der SEQ ID NO: 1, das Maltooligosyl-Trehalose-Synthase-Gen die Nukleotidsequenz der Nukleotide 601 bis 3033 der SEQ ID NO: 3 und das Maltooligosyl-Trehalose-Trehalohydrolase-Gen die Nukleotidsequenz der Nukleotide 601 bis 2430 der SEQ ID NO: 5 umfaßt.

6. Verfahren nach einem der Ansprüche 1 bis 4, wobei eines bzw. mehrere der Gene, ausgewählt aus der Gruppe:
6.1 für Aspartat-Kinase codierendes Gen,
6.2 für Dihydrodipicolinat-Synthase codierendes Gen,
6.3 für Glycerinaldehyd-3-phosphat-Dehydrogenase codierendes Gen,
6.4 für Pyruvat-Carboxylase codierendes Gen,
6.5 für Malat:Chinon-Oxidoreduktase codierendes Gen,
6.6 für Glucose-6-phosphat-Dehydrogenase codierendes Gen,
6.7 für ein Lysin exportierendes Protein codierendes Gen,
6.8 für das Protein Zwa1 codierendes Gen,
6.9 für Triosephosphat-Isomerase codierendes Gen und
6.10 für 3-Phosphoglycerat-Kinase codierendes Gen,
überexprimiert wird bzw. werden.

7. Verfahren nach einem der Ansprüche 1 bis 4, wobei eines bzw. mehrere der Gene, ausgewählt aus der Gruppe:
7.1 für Phosphoenolpyruvat-Carboxykinase codierendes Gen,
7.2 für Glucose-6-phosphat-Isomerase codierendes Gen,
7.3 für Pyruvat-Oxidase codierendes Gen,
7.4 für das Protein Zwa2 codierendes Gen,
7.5 für Homoserin-Dehydrogenase codierendes Gen,
7.6 für Homoserin-Kinase codierendes Gen,
ausgeschaltet ist bzw. sind.

8. Verfahren nach einem der Ansprüche 1 bis 4, wobei man zur Insertionsmutagenese des für Trehalose-Phosphatase codierenden Gens das unter DSM 14259 hinterlegte Plasmid pCR2.1otsBint verwendet.

9. Verfahren nach einem der Ansprüche 1 bis 4, wobei man zur Insertionsmutagenese des für Maltooligosyl-Trehalose-Synthase codierenden Gens das unter DSM 14260 hinterlegte Plasmid pCR2. 1treYint verwendet.

10. Verfahren nach einem der Ansprüche 1 bis 4, wobei man zur Insertionsmutagenese des für Maltooligosyl-Trehalose-Trehalohydrolase codierenden Gens das unter DSM 14261 hinterlegte Plasmid pCR2.1treZint verwendet.

## Revendications

1. Procédé pour la préparation fermentative de L-lysine qui comprend la mise en oeuvre des étapes suivantes :
a) fermentation d'une bactérie du genre *Corynebacterium* qui produit ladite L-lysine et dans laquelle au moins un gène choisi dans le groupe constitué par un gène codant la tréhalose phosphatase, un gène codant la maltooligosyl-tréhalose synthase et un gène codant la maltooligosyl-tréhalose tréhalohydrolase est éliminé,
b) concentration de la L-lysine dans le milieu ou dans les cellules de *Corynebacterium glutamicum,* et
c) isolement de la L-lysine facultativement avec les constituants du bouillon de fermentation et/ou de la biomasse restant en partie ou en quantité totale dans le produit final.

2. Procédé selon la revendication 1, dans lequel ladite bactérie du genre *Corynebacterium* est *Corynebacterium glutamicum.*

3. Procédé selon la revendication 1 ou 2, dans lequel ladite tréhalose phosphatase comprend la séquence d'acides aminés de SEQ ID n° 2, dans lequel ladite maltooligosyl-tréhalose synthase comprend la séquence d'acides aminés de SEQ ID n° 4, et dans lequel ladite maltooligosyl-tréhalose tréhalohydrolase comprend la séquence d'acides aminés de SEQ ID n° 6.

4. Procédé selon la revendication 1 ou 2, dans lequel ledit gène de la tréhalose phosphatase comprend la séquence nucléotidique de SEQ ID n° 1, dans lequel ledit gène de la maltooligosyl-tréhalose synthase comprend la séquence nucléotidique de SEQ ID n° 3 et dans lequel ledit gène de la maltooligosyl-tréhalose tréhalohydrolase comprend la séquence nucléotidique de SEQ ID n° 5.

5. Procédé selon la revendication 4, dans lequel ledit gène de la tréhalose phosphatase comprend la séquence nucléotidique composée des nucléotides 601 à 1368 de SEQ ID n° 1, dans lequel ledit gène de la maltooligosyl-tréhalose synthase comprend la séquence nucléotidique composée des nucléotides 601 à 3033 de SEQ ID n° 3 et dans lequel ledit gène de la maltooligosyl-tréhalose tréhalohydrolase comprend la séquence nucléotidique composée des nucléotides 601 à 2430 de SEQ ID n° 5.

6. Procédé selon l'une quelconque des revendications 1 à 4, dans lequel un ou plusieurs gènes choisi(s) dans le groupe constitué par :
6.1 le gène codant l'aspartate kinase,
6.2 le gène codant la dihydrodipicolinate synthase,
6.3 le gène codant la glycéraldéhyde 3-phosphate déshydrogénase,
6.4 le gène codant la pyruvate carboxylase,
6.5 le gène codant la malate quinone oxydoréductase,
6.6 le gène codant la glucose 6-phosphate déshydrogénase,
6.7 le gène codant une protéine qui exporte la lysine,
6.8 le gène codant la protéine Zwa1,
6.9 le gène codant la triose phosphate isomérase, et
6.10 le gène codant la 3-phosphoglycérate kinase,
est ou sont surexprimé(s).

7. Procédé selon l'une quelconque des revendications 1 à 4, dans lequel un ou plusieurs gènes choisi(s) dans le groupe constitué par :
7.1 le gène codant la phosphoénol pyruvate carboxykinase,
7.2 le gène codant la glucose 6-phosphate isomérase,
7.3 le gène codant la pyruvate oxydase,
7.4 le gène codant la protéine Zwa2,
7.5 le gène codant l'homosérine déshydrogénase,
7.6 le gène codant l'homosérine kinase,
est ou sont éliminé(s).

8. Procédé selon l'une quelconque des revendications 1 à 4, dans lequel pour la mutagenèse par insertion dudit gène codant la tréhalose phosphatase, le plasmide pCR2.1otsBint déposé sous le nom de DSM 14259 est utilisé.

9. Procédé selon l'une quelconque des revendications 1 à 4, dans lequel pour la mutagenèse par insertion dudit gène codant la maltooligosyl-tréhalose synthase, le plasmide pCR2.1treYint déposé sous le nom de DSM 14260 est utilisé.

10. Procédé selon l'une quelconque des revendications 1 à 4, dans lequel pour la mutagenèse par insertion dudit gène codant la maltooligosyl-tréhalose tréhalohydrolase, le plasmide pCR2.1treZint déposé sous le nom de DSM 14261 est utilisé.


### SEQUENCE PROTOCOL

<110> Degussa AG
<120> Process for the fermentative preparation of L-amino acids using coryneform bacteria
<130> 010133 BT
<140>
   <141>
<160> 12
<170> PatentIn Ver. 2.1
<210> 1
   <211> 1971
   <212> DNA
   <213> Corynebacterium glutamicum
<220>
   <221> CDS
   <222> (601)..(1368)
   <223> otsB gene
<400> 1
   cgtcaacgtg tgggctaata gtttcctgga ttgtttggca cagtcgggag aaaactcatg 60
   aaccgcgcac gaatcgcgac cataggcgtt cttccgcttg ctttactgct ggcgtcctgt 120
   ggttcagaca ccgtggaaat gacagattcc acctggttgg tgaccaatat ttacaccgat 180
   ccagatgagt cgaattcgat cagtaatctt gtcatttccc agcccagctt agattttggc 240
   aattcttccc tgtctggttt cactggctgt gtgcctttta cggggcgtgc ggaattcttc 300
   caaaatggtg agcaaagctc tgttctggat gccgattatg tgaccttgtc ttccctggat 360
   ttcgataaac ttcccgatga ttgccaagga caagaactca aagttcataa cgagctggtt 420
   gatcttctgc ctggttcttt tgaaatctcc aggacttctg gttcagaaat cttgctgact 480
   agcgatgtcg atgaactcga tcggccagca atccgcttgg tgtcctggat cgcgccgaca 540
   tcttaaggtg ccagggcttt aaagtgccag gggttctgtg ggatccgtac actggttccc 600
   atg act ttg act att gag gaa atc gcc aag acc aaa aag ctt ttg gtt    648
   Met Thr Leu Thr Ile Glu Glu Ile Ala Lys Thr Lys Lys Leu Leu Val
    1               5                   10                  15
   gtg tcc gat ttt gat gga acc atc gca gga ttt agc aag gac gct tac    696
   Val Ser Asp Phe Asp Gly Thr Ile Ala Gly Phe Ser Lys Asp Ala Tyr
               20                  25                  30
   aac gtt cct atc aac cag aaa tcc ctc aag gcg gta aaa gac ctc tcc    744
   Asn Val Pro Ile Asn Gln Lys Ser Leu Lys Ala Val Lys Asp Leu Ser
           35                  40                  45
   caa caa gca gac act gat gtt gtc att ttg tcg gga cgt cac ctg gag     792
   Gln Gln Ala Asp Thr Asp Val Val Ile Leu Ser Gly Arg His Leu Glu
       50                  55                  60
   gga ttg aag acg gtt ctt gat ctt ggt cag tac gac atc acc atg gtg     840
   Gly Leu Lys Thr Val Leu Asp Leu Gly Gln Tyr Asp Ile Thr Met Val
   65                  70                  75                  80
   ggt tca cac ggt tct gag gat tcc tcc cgc ccg cgt acc ctc act cct     888
   Gly Ser His Gly Ser Glu Asp Ser Ser Arg Pro Arg Thr Leu Thr Pro
                   85                  90                  95
   gaa gag gta gct cgc ctc gcc aag att gaa gca gat ctg gaa aag atc     936
   Glu Glu Val Ala Arg Leu Ala Lys Ile Glu Ala Asp Leu Glu Lys Ile
               100                 105                 110
   gtc gac ggc atc gaa ggc gca ttc gtg gag atc aag cct ttc cac cgc     984
   Val Asp Gly Ile Glu Gly Ala Phe Val Glu Ile Lys Pro Phe His Arg
           115                 120                 125
   gtg ctg cac ttc atc cgt gtt tcc gac aag gac aaa gtc caa gga atc    1032
   Val Leu His Phe Ile Arg Val Ser Asp Lys Asp Lys Val Gln Gly Ile
       130                 135                 140
   ctc gcc caa gca gca cac gta gac tct tcc ggc ctg aag gtt act aac    1080
   Leu Ala Gln Ala Ala His Val Asp Ser Ser Gly Leu Lys Val Thr Asn
   145                 150                 155                 160
   ggc aag agc atc atc gaa tac tcc atc agc tcc acc acc aag ggc acc    1128
   Gly Lys Ser Ile Ile Glu Tyr Ser Ile Ser Ser Thr Thr Lys Gly Thr
                   165                 170                 175
   tgg ctg aag gaa tac gtt gac cgc acc gag ccc act ggt gtg att ttc    1176
   Trp Leu Lys Glu Tyr Val Asp Arg Thr Glu Pro Thr Gly Val Ile Phe
               180                 185                 190
   ctc ggc gat gac acc acc gat gag cac ggt ttc aaa gct tta gaa aac    1224
   Leu Gly Asp Asp Thr Thr Asp Glu His Gly Phe Lys Ala Leu Glu Asn
           195                 200                 205
   gat gat cgt gcc cta acc gtc aag gtt ggc gaa gga gac act gca gcc    1272
   Asp Asp Arg Ala Leu Thr Val Lys Val Gly Glu Gly Asp Thr Ala Ala
       210                 215                 220
   aaa acc cgc gtc gac gat gtt gat aat gtg gga att ttc cta gag aaa    1320
   Lys Thr Arg Val Asp Asp Val Asp Asn Val Gly Ile Phe Leu Glu Lys
   225                 230                 235                 240
   ctc gcc tac cac cgc atg cag tat gcg gaa agc gtg cga ttg ggg att    1368
   Leu Ala Tyr His Arg Met Gln Tyr Ala Glu Ser Val Arg Leu Gly Ile
                   245                 250                 255
   taagagagcc taaacgcacg aaaagtgcca caagcctaga ttggcgcaac cgtggaacct 1428
   gggtggaagg aagtttccaa ttccacttct ggttccacgt attctttgtc aatcattttc 1488
   aacagtgttt cgccggcttt gaaccctttg agtttgttgg gttggatgac ggtggtgaga 1548
   tcccgtgcga gtgccatgtg ggtgccatcg aaaccagtga gggatagatc tgcaggcgct 1608
   gatttaccta cgcttttaag gtattccaga acgccgaatg ccagtgcatc gacggtacag 1668
   agtactgcgg tgaggtctgg gtgtgtttct agaagttctt tggccacttc gaagttgtgt 1728
   tggcggttgt tgatccagca ttccatgatc ggcacggtgc cgggatcgat tcccgcttcg 1788
   ataaagactt ccatggcacc cctgactcgg tcgcgttgta cctggtattg ggcgttttcg 1848
   aggcgctcgc gggtgacttc gccgtcgttg tttgcgcggt ctaggcggat ggacaggatg 1908
   ccgattttgc ggtggccggc gtcgattagc gcttgagctg caggggcgat ggctttgcgg 1968
   tta 1971
<210> 2
   <211> 256
   <212> PRT
   <213> Corynebacterium glutamicum
<400> 2
   Met Thr Leu Thr Ile Glu Glu Ile Ala Lys Thr Lys Lys Leu Leu Val
   1 5 10 15
   Val Ser Asp Phe Asp Gly Thr Ile Ala Gly Phe Ser Lys Asp Ala Tyr
   20 25 30
   Asn Val Pro Ile Asn Gln Lys Ser Leu Lys Ala Val Lys Asp Leu Ser
   35 40 45
   Gln Gln Ala Asp Thr Asp Val Val Ile Leu Ser Gly Arg His Leu Glu
   50 55 60
   Gly Leu Lys Thr Val Leu Asp Leu Gly Gln Tyr Asp Ile Thr Met Val
   65 70 75 80
   Gly Ser His Gly Ser Glu Asp Ser Ser Arg Pro Arg Thr Leu Thr Pro
   85 90 95
   Glu Glu Val Ala Arg Leu Ala Lys Ile Glu Ala Asp Leu Glu Lys Ile
   100 105 110
   Val Asp Gly Ile Glu Gly Ala Phe Val Glu Ile Lys Pro Phe His Arg
   115 120 125
   Val Leu His Phe Ile Arg Val Ser Asp Lys Asp Lys Val Gln Gly Ile
   130 135 140
   Leu Ala Gln Ala Ala His Val Asp Ser Ser Gly Leu Lys Val Thr Asn
   145 150 155 160
   Gly Lys Ser Ile Ile Glu Tyr Ser Ile Ser Ser Thr Thr Lys Gly Thr
   165 170 175
   Trp Leu Lys Glu Tyr Val Asp Arg Thr Glu Pro Thr Gly Val Ile Phe
   180 185 190
   Leu Gly Asp Asp Thr Thr Asp Glu His Gly Phe Lys Ala Leu Glu Asn
           195                 200                 205
   Asp Asp Arg Ala Leu Thr Val Lys Val Gly Glu Gly Asp Thr Ala Ala
       210                 215                 220
   Lys Thr Arg Val Asp Asp Val Asp Asn Val Gly Ile Phe Leu Glu Lys
   225                 230                 235                 240
   Leu Ala Tyr His Arg Met Gln Tyr Ala Glu Ser Val Arg Leu Gly Ile
                   245                 250                 255
<210> 3
   <211> 3636
   <212> DNA
   <213> Corynebacterium glutamicum
<220>
   <221> CDS
   <222> (601)..(3033)
   <223> treY gene
<400> 3
   ataacccagc aatgctgaac ctttcccacg atctgtgtat ggggttgcac agcgggaaag 60
   tcacactcag ttaccgggct ctcgctgaag tggggctgac caccaggcaa gcgtgggatc 120
   aatcagccac caatttatta aagtgtgcta ctactcctga gggtattcga tttgatcttc 180
   gggacgcaga aacgaccaca accatcgcct cgtctgcatt agaaatcaga gttcccggcg 240
   cgccaattac tgcatggttg gcgcatcctc aaacctttac agtgttgaat cgtcatcttg 300
   aattacgact gggaccagca ccgctgtatc ttgctccgaa ttcacacaca ctcatcgcta 360
   ttcctgctgg tgatccaggg atctttgaat ttgaacggtg ggcgcgaagc ttgctggaag 420
   tgggtggtgt ggagggtatc gtcgataagc ttcttgttta taggcacggc tttccttgcc 480
   cttaccagcc agcctttgta gcacttgctg cgtaaatctt tttcccacgc cgggaatgcg 540
   tgaacactaa gatcgaggac gtaccgcacg attttgccta acttttaagg gtgtttcatc 600
   atg gca cgt cca att tcc gca acg tac agg ctt caa atg cga gga cct   648
   Met Ala Arg Pro Ile Ser Ala Thr Tyr Arg Leu Gln Met Arg Gly Pro
   1               5                   10                  15
   caa gca gat agc gcc ggg cgt tca ttt ggt ttt gcg cag gcc aaa gcc   696
   Gln Ala Asp Ser Ala Gly Arg Ser Phe Gly Phe Ala Gln Ala Lys Ala
               20                  25                  30
   cag ctt ccc tat ctg aag aag cta ggc atc agc cac ctg tac ctc tcc   744
   Gln Leu Pro Tyr Leu Lys Lys Leu Gly Ile Ser His Leu Tyr Leu Ser
           35                  40                  45
   cct att ttt acg gcc atg cca gat tcc aat cat ggc tac gat gtc att     792
   Pro Ile Phe Thr Ala Met Pro Asp Ser Asn His Gly Tyr Asp Val Ile
        50                  55                  60
   gat ccc acc acc atc aat gaa gag ctc ggt ggc atg gag ggt ctt cga     840
   Asp Pro Thr Thr Ile Asn Glu Glu Leu Gly Gly Met Glu Gly Leu Arg
   65                  70                  75                  80
   gat ctt gcc gca gct aca cac gag ttg ggc atg ggc atc atc att gat     888
   Asp Leu Ala Ala Ala Thr His Glu Leu Gly Met Gly Ile Ile Ile Asp
                   85                  90                  95
   att gtt ccc aac cat tta ggt gtt gcc gtt cca cat ttg aat cct tgg     936
   Ile Val Pro Asn His Leu Gly Val Ala Val Pro His Leu Asn Pro Trp
               100                 105                 110
   tgg tgg gat gtt cta aaa aac ggc aaa gat tcc gct ttt gag ttc tat     984
   Trp Trp Asp Val Leu Lys Asn Gly Lys Asp Ser Ala Phe Glu Phe Tyr
           115                 120                 125
   ttc gat att gac tgg cac gaa gac aac ggt tct ggt ggc aag ctg ggc    1032
   Phe Asp Ile Asp Trp His Glu Asp Asn Gly Ser Gly Gly Lys Leu Gly
       130                 135                 140
   atg ccg att ctg ggt gct gaa ggc gat gaa gac aag ctg gaa ttc gcg    1080
   Met Pro Ile Leu Gly Ala Glu Gly Asp Glu Asp Lys Leu Glu Phe Ala
   145                 150                 155                 160
   gag ctt gat gga gag aaa gtg ctc aaa tat ttt gac cac ctc ttc cca    1128
   Glu Leu Asp Gly Glu Lys Val Leu Lys Tyr Phe Asp His Leu Phe Pro
                   165                 170                 175
   atc gcg cct ggt acc gaa gaa ggg aca ccg caa gaa gtc tac aag cgc    1176
   Ile Ala Pro Gly Thr Glu Glu Gly Thr Pro Gln Glu Val Tyr Lys Arg
               180                 185                 190
   cag cat tac cgc ctg cag ttc tgg cgc gat ggc gtg atc aac ttc cgt    1224
   Gln His Tyr Arg Leu Gln Phe Trp Arg Asp Gly Val Ile Asn Phe Arg
           195                 200                 205
   cgc ttc ttt tcc gtg aat acg ttg gct ggc atc agg caa gaa gat ccc    1272
   Arg Phe Phe Ser Val Asn Thr Leu Ala Gly Ile Arg Gln Glu Asp Pro
       210                 215                 220
   tta gtg ttt gaa cat act cat cgt ctg ctg cgc gaa ttg gtg gcg gaa    1320
   Leu Val Phe Glu His Thr His Arg Leu Leu Arg Glu Leu Val Ala Glu
   225                 230                 235                 240
   gac ctc att gac ggc gtg cgc gtc gat cac ccc gac ggg ctt tcc gat    1368
   Asp Leu Ile Asp Gly Val Arg Val Asp His Pro Asp Gly Leu Ser Asp
                   245                 250                 255
   cct ttt gga tat ctg cac aga ctc cgc gac ctc att gga cct gac cgc    1416
   Pro Phe Gly Tyr Leu His Arg Leu Arg Asp Leu Ile Gly Pro Asp Arg
               260                 265                 270
   tgg ctg atc atc gaa aag atc ttg agc gtt gat gaa cca ctc gat ccc    1464
   Trp Leu Ile Ile Glu Lys Ile Leu Ser Val Asp Glu Pro Leu Asp Pro
           275                 280                 285
   cgc ctg gcc gtt gat ggc acc act ggc tac gac gcc ctc cgt gaa ctc     1512
   Arg Leu Ala Val Asp Gly Thr Thr Gly Tyr Asp Ala Leu Arg Glu Leu
       290                 295                 300
   gac ggc gtg ttt atc tcc cga gaa tct gag gac aaa ttc tcc atg ctg     1560
   Asp Gly Val Phe Ile Ser Arg Glu Ser Glu Asp Lys Phe Ser Met Leu
   305                 310                 315                 320
   gcg ctg acc cac agt gga tcc acc tgg gat gaa cgc gcc ctc aaa tcc     1608
   Ala Leu Thr His Ser Gly Ser Thr Trp Asp Glu Arg Ala Leu Lys Ser
                   325                 330                 335
   acg gag gaa agc ctc aaa cga gtc gtc gcc caa caa gaa ctc gca gcc     1656
   Thr Glu Glu Ser Leu Lys Arg Val Val Ala Gln Gln Glu Leu Ala Ala
               340                 345                 350
   gaa atc tta agg ctc gcc cgc gcc atg cgc cgc gat aac ttc tcc acc     1704
   Glu Ile Leu Arg Leu Ala Arg Ala Met Arg Arg Asp Asn Phe Ser Thr
           355                 360                 365
   gca ggc acc aac gtc acc gaa gac aaa ctt agc gaa acc atc atc gaa     1752
   Ala Gly Thr Asn Val Thr Glu Asp Lys Leu Ser Glu Thr Ile Ile Glu
       370                 375                 380
   tta gtc gcc gcc atg ccc gtc tac cgc gcc gac tac atc tcc ctc tca     1800
   Leu Val Ala Ala Met Pro Val Tyr Arg Ala Asp Tyr Ile Ser Leu Ser
   385                 390                 395                 400
   cgc acc acc gcc acc gtc atc gcg gag atg tcc aaa cgc ttc ccc tcc     1848
   Arg Thr Thr Ala Thr Val Ile Ala Glu Met Ser Lys Arg Phe Pro Ser
                   405                 410                 415
   cgg cgt gac gca ctc gac ctc atc gcg gcc gcc cta ctt ggc aat ggc     1896
   Arg Arg Asp Ala Leu Asp Leu Ile Ala Ala Ala Leu Leu Gly Asn Gly
               420                 425                 430
   gag gcc aaa atc cgc ttc gct caa gtc tgc ggc gcc gtc atg gct aaa     1944
   Glu Ala Lys Ile Arg Phe Ala Gln Val Cys Gly Ala Val Met Ala Lys
           435                 440                 445
   ggt gtg gaa gac acc acc ttc tac cgc gca tct agg ctc gtt gca ttg     1992
   Gly Val Glu Asp Thr Thr Phe Tyr Arg Ala Ser Arg Leu Val Ala Leu
       450                 455                 460
   caa gaa gtc ggt ggc gcg ccg ggg aga ttc ggc gtc tcc gct gca gaa     2040
   Gln Glu Val Gly Gly Ala Pro Gly Arg Phe Gly Val Ser Ala Ala Glu
   465                 470                 475                 480
   ttc cac ttg ctg cag gaa gaa cgc agc ctg ctg tgg cca cgc acc atg     2088
   Phe His Leu Leu Gln Glu Glu Arg Ser Leu Leu Trp Pro Arg Thr Met
                   485                 490                 495
   acc acc ttg tcc acg cat gac acc aaa cgt ggc gaa gat acc cgc gcc     2136
   Thr Thr Leu Ser Thr His Asp Thr Lys Arg Gly Glu Asp Thr Arg Ala
               500                 505                 510
   cgc atc atc tcc ctg tct gaa gtc ccc gat atg tac tcc gag ctg gtc    2184
   Arg Ile Ile Ser Leu Ser Glu Val Pro Asp Met Tyr Ser Glu Leu Val
           515                 520                 525
   aat cgt gtt ttc gcg gtg ctc ccc gcg cca gac ggc gca acg ggc agt    2232
   Asn Arg Val Phe Ala Val Leu Pro Ala Pro Asp Gly Ala Thr Gly Ser
       530                 535                 540
   ttc ctc cta caa aac ctg ctg ggc gta tgg ccc gcc gac ggc gtg atc    2280
   Phe Leu Leu Gln Asn Leu Leu Gly Val Trp Pro Ala Asp Gly Val Ile
   545                 550                 555                 560
   acc gat gcg ctg cgc gat cga ttc agg gaa tac gcc cta aaa gct atc    2328
   Thr Asp Ala Leu Arg Asp Arg Phe Arg Glu Tyr Ala Leu Lys Ala Ile
                   565                 570                 575
   cgc gaa gca tcc aca aaa acc acg tgg gtg gac ccc aac gag tcc ttc    2376
   Arg Glu Ala Ser Thr Lys Thr Thr Trp Val Asp Pro Asn Glu Ser Phe
               580                 585                 590
   gag gct gcg gtc tgc gat tgg gtg gaa gcg ctt ttc gac gga ccc tcc    2424
   Glu Ala Ala Val Cys Asp Trp Val Glu Ala Leu Phe Asp Gly Pro Ser
           595                 600                 605
   acc tca cta atc acc gaa ttt gtc tcc cac atc aac cgt ggc tct gtg    2472
   Thr Ser Leu Ile Thr Glu Phe Val Ser His Ile Asn Arg Gly Ser Val
       610                 615                 620
   caa atc tcc tta ggc agg aaa ctg ctg caa atg gtg ggc gct gga atc    2520
   Gln Ile Ser Leu Gly Arg Lys Leu Leu Gln Met Val Gly Ala Gly Ile
   625                 630                 635                 640
   ccc gac act tac caa gga act gag ttt tta gaa gac tcc ctg gta gat    2568
   Pro Asp Thr Tyr Gln Gly Thr Glu Phe Leu Glu Asp Ser Leu Val Asp
                   645                 650                 655
   ccc gat aac cga cgc ttt gtt gat tac acc gcc aga gaa caa gtc ctg    2616
   Pro Asp Asn Arg Arg Phe Val Asp Tyr Thr Ala Arg Glu Gln Val Leu
               660                 665                 670
   gag cgc ctg caa acc tgg gat tgg acg cag gtt aat tcg gta gaa gac    2664
   Glu Arg Leu Gln Thr Trp Asp Trp Thr Gln Val Asn Ser Val Glu Asp
           675                 680                 685
   ttg gtg gat aac gcc gac atc gcc aaa atg gcc gtg gtc cat aaa tcc    2712
   Leu Val Asp Asn Ala Asp Ile Ala Lys Met Ala Val Val His Lys Ser
       690                 695                 700
   ctc gag ttg cgt gct gaa ttt cgt gca agc ttt gtt ggt gga gat cat    2760
   Leu Glu Leu Arg Ala Glu Phe Arg Ala Ser Phe Val Gly Gly Asp His
   705                 710                 715                 720
   cag gca gta ttt ggc gaa ggt cgc gca gaa tcc cac atc atg ggc atc    2808
   Gln Ala Val Phe Gly Glu Gly Arg Ala Glu Ser His Ile Met Gly Ile
                   725                 730                 735
   gcc cgc ggt aca gac cga aac cac ctc aac atc att gct ctt gct acc    2856
   Ala Arg Gly Thr Asp Arg Asn His Leu Asn Ile Ile Ala Leu Ala Thr
               740                 745                 750
   cgt cga cca ctg atc ttg gaa gac cgt ggc gga tgg tat gac acc acc   2904
   Arg Arg Pro Leu Ile Leu Glu Asp Arg Gly Gly Trp Tyr Asp Thr Thr
           755                 760                 765
   gtc acg ctt cct ggt gga caa tgg gaa gac agg ctc acc ggg caa cgc   2952
   Val Thr Leu Pro Gly Gly Gln Trp Glu Asp Arg Leu Thr Gly Gln Arg
       770                 775                 780
   ttc agt ggt gtt gtc cca gcc acc gat ttg ttc tca cat cta ccc gta   3000
   Phe Ser Gly Val Val Pro Ala Thr Asp Leu Phe Ser His Leu Pro Val
   785                 790                 795                 800
   tct ttg ttg gtt tta gta ccc gat agt gag ttt tgatccctgc acaggaaagt 3053
   Ser Leu Leu Val Leu Val Pro Asp Ser Glu Phe
                   805                 810
   tagcggcgct actatgaacg atcgatatgt ctgacaacac tctctcccaa tttggcagtt 3113
   actaccacga attccgacgt gcccatccca tggccgacgt cgaattcctc ctagcaattg 3173
   aagaattact tacggacggt ggtgtcacct tcgatcgcgt caccacacgc atcaaagaat 3233
   ggtcaagcct gaaagccaag gctcgcaagc gtcgcaacga tggctcgttg atctaccctg 3293
   atccgcgcaa agacatccac gacatgatcg gtgttcggat caccacgtac cactccacgg 3353
   aaatacccgt ggccctaaaa gtgctccaag actccttcat cgtccacaaa tccgtagaca 3413
   aagccgctga aactcgcatc tcaggcggct ttggttacgg ctcccaccac ctgattctgg 3473
   aagtcgatga cacctccgat gacctccagg actacaaagg cctcgtcttt gaagttcagg 3533
   tgcgcaccgt gctgcaacac gcctgggcag agttcgaaca cgatatccgc tataaacgcg 3593
   ccgatgtgtc caacccagaa gacttcagcg cagaagtaga ccg                   3636
<210> 4
   <211> 811
   <212> PRT
   <213> Corynebacterium glutamicum
<400> 4
   Met Ala Arg Pro Ile Ser Ala Thr Tyr Arg Leu Gln Met Arg Gly Pro
   1               5                   10                  15
   Gln Ala Asp Ser Ala Gly Arg Ser Phe Gly Phe Ala Gln Ala Lys Ala
               20                  25                  30
   Gln Leu Pro Tyr Leu Lys Lys Leu Gly Ile Ser His Leu Tyr Leu Ser
           35                  40                  45
   Pro Ile Phe Thr Ala Met Pro Asp Ser Asn His Gly Tyr Asp Val Ile
       50                  55                  60
   Asp Pro Thr Thr Ile Asn Glu Glu Leu Gly Gly Met Glu Gly Leu Arg
   65                  70                  75                  80
   Asp Leu Ala Ala Ala Thr His Glu Leu Gly Met Gly Ile Ile Ile Asp
                   85                  90                  95
   Ile Val Pro Asn His Leu Gly Val Ala Val Pro His Leu Asn Pro Trp
               100                 105                 110
   Trp Trp Asp Val Leu Lys Asn Gly Lys Asp Ser Ala Phe Glu Phe Tyr
           115                 120                 125
   Phe Asp Ile Asp Trp His Glu Asp Asn Gly Ser Gly Gly Lys Leu Gly
       130                 135                 140
   Met Pro Ile Leu Gly Ala Glu Gly Asp Glu Asp Lys Leu Glu Phe Ala
   145                 150                 155                 160
   Glu Leu Asp Gly Glu Lys Val Leu Lys Tyr Phe Asp His Leu Phe Pro
                   165                 170                 175
   Ile Ala Pro Gly Thr Glu Glu Gly Thr Pro Gln Glu Val Tyr Lys Arg
               180                 185                 190
   Gln His Tyr Arg Leu Gln Phe Trp Arg Asp Gly Val Ile Asn Phe Arg
           195                 200                 205
   Arg Phe Phe Ser Val Asn Thr Leu Ala Gly Ile Arg Gln Glu Asp Pro
       210                 215                 220
   Leu Val Phe Glu His Thr His Arg Leu Leu Arg Glu Leu Val Ala Glu
   225                 230                 235                 240
   Asp Leu Ile Asp Gly Val Arg Val Asp His Pro Asp Gly Leu Ser Asp
                   245                 250                 255
   Pro Phe Gly Tyr Leu His Arg Leu Arg Asp Leu Ile Gly Pro Asp Arg
               260                 265                 270
   Trp Leu Ile Ile Glu Lys Ile Leu Ser Val Asp Glu Pro Leu Asp Pro
           275                 280                 285
   Arg Leu Ala Val Asp Gly Thr Thr Gly Tyr Asp Ala Leu Arg Glu Leu
       290                 295                 300
   Asp Gly Val Phe Ile Ser Arg Glu Ser Glu Asp Lys Phe Ser Met Leu
   305                 310                 315                 320
   Ala Leu Thr His Ser Gly Ser Thr Trp Asp Glu Arg Ala Leu Lys Ser
                   325                 330                 335
   Thr Glu Glu Ser Leu Lys Arg Val Val Ala Gln Gln Glu Leu Ala Ala
               340                 345                 350
   Glu Ile Leu Arg Leu Ala Arg Ala Met Arg Arg Asp Asn Phe Ser Thr
           355                 360                 365
   Ala Gly Thr Asn Val Thr Glu Asp Lys Leu Ser Glu Thr Ile Ile Glu
       370                 375                 380
   Leu Val Ala Ala Met Pro Val Tyr Arg Ala Asp Tyr Ile Ser Leu Ser
   385                 390                 395                 400
   Arg Thr Thr Ala Thr Val Ile Ala Glu Met Ser Lys Arg Phe Pro Ser
   405 410 415
   Arg Arg Asp Ala Leu Asp Leu Ile Ala Ala Ala Leu Leu Gly Asn Gly
   420 425 430
   Glu Ala Lys Ile Arg Phe Ala Gln Val Cys Gly Ala Val Met Ala Lys
   435 440 445
   Gly Val Glu Asp Thr Thr Phe Tyr Arg Ala Ser Arg Leu Val Ala Leu
   450 455 460
   Gln Glu Val Gly Gly Ala Pro Gly Arg Phe Gly Val Ser Ala Ala Glu
   465 470 475 480
   Phe His Leu Leu Gln Glu Glu Arg Ser Leu Leu Trp Pro Arg Thr Met
   485 490 495
   Thr Thr Leu Ser Thr His Asp Thr Lys Arg Gly Glu Asp Thr Arg Ala
   500 505 510
   Arg Ile Ile Ser Leu Ser Glu Val Pro Asp Met Tyr Ser Glu Leu Val
   515 520 525
   Asn Arg Val Phe Ala Val Leu Pro Ala Pro Asp Gly Ala Thr Gly Ser
   530 535 540
   Phe Leu Leu Gln Asn Leu Leu Gly Val Trp Pro Ala Asp Gly Val Ile
   545 550 555 560
   Thr Asp Ala Leu Arg Asp Arg Phe Arg Glu Tyr Ala Leu Lys Ala Ile
   565 570 575
   Arg Glu Ala Ser Thr Lys Thr Thr Trp Val Asp Pro Asn Glu Ser Phe
   580 585 590
   Glu Ala Ala Val Cys Asp Trp Val Glu Ala Leu Phe Asp Gly Pro Ser
   595 600 605
   Thr Ser Leu Ile Thr Glu Phe Val Ser His Ile Asn Arg Gly Ser Val
   610 615 620
   Gln Ile Ser Leu Gly Arg Lys Leu Leu Gln Met Val Gly Ala Gly Ile
   625 630 635 640
   Pro Asp Thr Tyr Gln Gly Thr Glu Phe Leu Glu Asp Ser Leu Val Asp
   645 650 655
   Pro Asp Asn Arg Arg Phe Val Asp Tyr Thr Ala Arg Glu Gln Val Leu
   660 665 670
   Glu Arg Leu Gln Thr Trp Asp Trp Thr Gln Val Asn Ser Val Glu Asp
   675 680 685
   Leu Val Asp Asn Ala Asp Ile Ala Lys Met Ala Val Val His Lys Ser
   690 695 700
   Leu Glu Leu Arg Ala Glu Phe Arg Ala Ser Phe Val Gly Gly Asp His
   705                 710                 715                 720
   Gln Ala Val Phe Gly Glu Gly Arg Ala Glu Ser His Ile Met Gly Ile
                   725                 730                 735
   Ala Arg Gly Thr Asp Arg Asn His Leu Asn Ile Ile Ala Leu Ala Thr
               740                 745                 750
   Arg Arg Pro Leu Ile Leu Glu Asp Arg Gly Gly Trp Tyr Asp Thr Thr
           755                 760                 765
   Val Thr Leu Pro Gly Gly Gln Trp Glu Asp Arg Leu Thr Gly Gln Arg
       770                 775                 780
   Phe Ser Gly Val Val Pro Ala Thr Asp Leu Phe Ser His Leu Pro Val
   785                 790                 795                 800
   Ser Leu Leu Val Leu Val Pro Asp Ser Glu Phe
                   805                 810
<210> 5
   <211> 3033
   <212> DNA
   <213> Corynebacterium glutamicum
<220>
   <221> CDS
   <222> (601)..(2430)
   <223> treZ gene
<400> 5
   ctagtcactc ggggattcca cccacatgtg cccggattct ttcaattcaa tttctgcacc 60
   catcatggat gacaattgag attccaccgc agcctgttca ccaggcaata aagccaaggt 120
   gtatgtgact tctgcaccat attcagtgtc agtaatgatg atgcccatgc cgcgcagatt 180
   cgcttcaatg cgccccgcat cagaatgcgg aaggtcaatc ttgaaaatct cccgaacaga 240
   gcgggtgact tgcaaaacct caggtagaag ctccgtcacc gcgttggtgt aggcattaac 300
   taatccgcca gtgcccagtt ttacgccacc gaaataacgc accaccacag cggcaatatc 360
   tttcattccc gagccacgca acgcctctag catgggtttt cccgcggtac cggaaggttc 420
   gccatcatca gaggaacgct ccacatcatt tgagccatcc acatggaaaa taaaggcact 480
   gcaatgatga cgcgcatccg gatacagctc cttgatggag tgaataaatt cgcgagcctg 540
   ctcctgatct tgcacacgcg tgatataggt cagaaatcgc gagcgcttga tctctagttc 600
   atg ctc aaa gac ttg acc ggc ctg agg gag ttg gta ttg cgt gag atg   648
   Met Leu Lys Asp Leu Thr Gly Leu Arg Glu Leu Val Leu Arg Glu Met
    1               5                  10                  15
   tgc cat agc atc tca cat ctt agc tcg cca acc ggc agc att ttc act    696
   Cys His Ser Ile Ser His Leu Ser Ser Pro Thr Gly Ser Ile Phe Thr
               20                  25                  30
   agc ctg gtg gcc atg ttg acc tcg caa agc ttt tca gtg tgg gct cca    744
   Ser Leu Val Ala Met Leu Thr Ser Gln Ser Phe Ser Val Trp Ala Pro
           35                  40                  45
   ctt ccc cac gat gta cat ctg atc ctc aac ggc gaa acc ctc ccc atg    792
   Leu Pro His Asp Val His Leu Ile Leu Asn Gly Glu Thr Leu Pro Met
       50                  55                  60
   cac aaa acg gag ggc agc tgg tgg cgc gcc gag atc gcg ccc aag gcc    840
   His Lys Thr Glu Gly Ser Trp Trp Arg Ala Glu Ile Ala Pro Lys Ala
   65                  70                  75                  80
   ggc gat cgt tac ggt ttt tcg ctt ttc gac ggc tcc tcc tgg tca aaa    888
   Gly Asp Arg Tyr Gly Phe Ser Leu Phe Asp Gly Ser Ser Trp Ser Lys
                   85                  90                  95
   acc ctc ccc gat ccc cgc tcc aca tct caa cca gac ggg gtt cat ggt    936
   Thr Leu Pro Asp Pro Arg Ser Thr Ser Gln Pro Asp Gly Val His Gly
               100                 105                 110
   tta agt gaa gtc tcc gat gat tcc tat ctg tgg ggt gac cag cag tgg    984
   Leu Ser Glu Val Ser Asp Asp Ser Tyr Leu Trp Gly Asp Gln Gln Trp
           115                 120                 125
   act ggc cga att ctc cct ggc tcg gtg tta tat gag ctg cat gtg ggc    1032
   Thr Gly Arg Ile Leu Pro Gly Ser Val Leu Tyr Glu Leu His Val Gly
       130                 135                 140
   acc ttt agt gaa gat gga acg ttt gag gga gtc gtc gac aag ctt cct    1080
   Thr Phe Ser Glu Asp Gly Thr Phe Glu Gly Val Val Asp Lys Leu Pro
   145                 150                 155                 160
   tat ctg cgc gac ctc ggc gtg acc gcc atc gaa ctt tta ccc gtg cag    1128
   Tyr Leu Arg Asp Leu Gly Val Thr Ala Ile Glu Leu Leu Pro Val Gln
                   165                 170                 175
   ccc ttt ggc ggc aac cgc aat tgg ggc tac gac ggg gtg ctg tgg cac    1176
   Pro Phe Gly Gly Asn Arg Asn Trp Gly Tyr Asp Gly Val Leu Trp His
               180                 185                 190
   gcc gtc cat gca ggc tac ggc ggt ccg gcg ggc ttg aaa aag ctt atc    1224
   Ala Val His Ala Gly Tyr Gly Gly Pro Ala Gly Leu Lys Lys Leu Ile
           195                 200                 205
   gac gcc tcc cac cag gcc ggc atc gcc gtc tac tta gac gtc gtg tac    1272
   Asp Ala Ser His Gln Ala Gly Ile Ala Val Tyr Leu Asp Val Val Tyr
       210                 215                 220
   aac cac ttc ggc ccc gac ggc aac tac aac ggg caa ttt ggc ccc tac    1320
   Asn His Phe Gly Pro Asp Gly Asn Tyr Asn Gly Gln Phe Gly Pro Tyr
   225                 230                 235                 240
   acc tct ggc ggc agc acc ggc tgg ggc gac gtg gtc aac atc aac ggc    1368
   Thr Ser Gly Gly Ser Thr Gly Trp Gly Asp Val Val Asn Ile Asn Gly
                   245                 250                 255
   cat gat tca gat gaa gtc cgc aat tat att ctc gac gcc gca cgc cag    1416
   His Asp Ser Asp Glu Val Arg Asn Tyr Ile Leu Asp Ala Ala Arg Gln
               260                 265                 270
   tgg ttc gaa gat ttt cac gtt gat ggg ctc cgc ctc gat gcg gtg cat    1464
   Trp Phe Glu Asp Phe His Val Asp Gly Leu Arg Leu Asp Ala Val His
           275                 280                 285
   tct ctc gat gat cgc ggc gcc tat tcc cta ctt gcg cag ctg acc atg    1512
   Ser Leu Asp Asp Arg Gly Ala Tyr Ser Leu Leu Ala Gln Leu Thr Met
       290                 295                 300
   gtg gcc gag gat gtc tcc gca caa aca ggc atc cca cgc tca ttg att    1560
   Val Ala Glu Asp Val Ser Ala Gln Thr Gly Ile Pro Arg Ser Leu Ile
   305                 310                 315                 320
   gca gaa tct gaa ctc aat gac ccc aag ttc gtt acc tcc cgc gag gcc    1608
   Ala Glu Ser Glu Leu Asn Asp Pro Lys Phe Val Thr Ser Arg Glu Ala
                   325                 330                 335
   ggc ggt ttt ggc ctg gat gca cag tgg gtt gac gat atc cac cac gcc    1656
   Gly Gly Phe Gly Leu Asp Ala Gln Trp Val Asp Asp Ile His His Ala
               340                 345                 350
   ctc cat gcc ctc gtt tct ggc gaa cgc aat ggt tat tac agc gat ttc    1704
   Leu His Ala Leu Val Ser Gly Glu Arg Asn Gly Tyr Tyr Ser Asp Phe
           355                 360                 365
   gga tct gtc gac aca tta gcc aaa acc ctg cgt gaa gta ttt gaa cac    1752
   Gly Ser Val Asp Thr Leu Ala Lys Thr Leu Arg Glu Val Phe Glu His
       370                 375                 380
   acc gga aac tac tcc acg tac cgc gga cgc aac cac ggc cgc cct gtg    1800
   Thr Gly Asn Tyr Ser Thr Tyr Arg Gly Arg Asn His Gly Arg Pro Val
   385                 390                 395                 400
   cac ccc gat atc acc cct gcc tcg cgc ttt gtc acc tac acc acc acc    1848
   His Pro Asp Ile Thr Pro Ala Ser Arg Phe Val Thr Tyr Thr Thr Thr
                   405                 410                 415
   cat gat cag acc ggc aac cgc gca atc ggc gac cgt cct tcc acg act    1896
   His Asp Gln Thr Gly Asn Arg Ala Ile Gly Asp Arg Pro Ser Thr Thr
               420                 425                 430
   ctc acc ccg gaa cag cag gtg ttg aag gca gcc att atc tac agc tcg    1944
   Leu Thr Pro Glu Gln Gln Val Leu Lys Ala Ala Ile Ile Tyr Ser Ser
           435                 440                 445
   ccg tat acc ccg atg ttg ttt atg ggt gaa gaa ttc gga gcc acc acc    1992
   Pro Tyr Thr Pro Met Leu Phe Met Gly Glu Glu Phe Gly Ala Thr Thr
       450                 455                 460
   cca ttc gcc ttc ttt tgc tcc cac acc gac ccc gag ctc aac cgc cta    2040
   Pro Phe Ala Phe Phe Cys Ser His Thr Asp Pro Glu Leu Asn Arg Leu
   465                 470                 475                 480
   acc tcc gag ggc cgc aaa cgg gaa ttc gca cgc ctt ggc tgg aac gcc    2088
   Thr Ser Glu Gly Arg Lys Arg Glu Phe Ala Arg Leu Gly Trp Asn Ala
                   485                 490                 495
   gac gac atc ccc tcc ccc gag ctg gaa tcc acc ttc acc tcc tcc aaa    2136
   Asp Asp Ile Pro Ser Pro Glu Leu Glu Ser Thr Phe Thr Ser Ser Lys
               500                 505                 510
   ctc gat tgg gag ttc act gcg gag cag cgc cgc atc aac gac gct tac    2184
   Leu Asp Trp Glu Phe Thr Ala Glu Gln Arg Arg Ile Asn Asp Ala Tyr
           515                 520                 525
   aag cag ctg ttg cac ctg cgg cac acc ttg ggc ttc tcc caa cca aac    2232
   Lys Gln Leu Leu His Leu Arg His Thr Leu Gly Phe Ser Gln Pro Asn
       530                 535                 540
   ttg ctc aca ctc gag gtt gag cac ggc gag aac tgg cta tcg atg gcc    2280
   Leu Leu Thr Leu Glu Val Glu His Gly Glu Asn Trp Leu Ser Met Ala
   545                 550                 555                 560
   aat ggt cgc ggc cga att ctg gcg aat ttc tcc gac gac acc atc acc    2328
   Asn Gly Arg Gly Arg Ile Leu Ala Asn Phe Ser Asp Asp Thr Ile Thr
                   565                 570                 575
   gtc ccg ctt ggc ggc gag ctg att tac agc ttc act tcc ccc acc gtc    2376
   Val Pro Leu Gly Gly Glu Leu Ile Tyr Ser Phe Thr Ser Pro Thr Val
               580                 585                 590
   acc gac acc tcc aca acc ctt cag ccg tgg ggc ttt gcg atc ctg acc    2424
   Thr Asp Thr Ser Thr Thr Leu Gln Pro Trp Gly Phe Ala Ile Leu Thr
           595                 600                 605
   cga aac tagaaaaagg ccacctcgat tgaggtggcc ttcagctatg tttaggtcaa    2480
   Arg Asn
       610
   gtattcgtac tcaggggtgc ccggctcgag gcgacgggaa tcaattgccg attcctccat 2540
   acgttccagc aaagaatcca atcctgatgc ttcactcaag tgaataccca ccaacgcagt 2600
   accggtctca cggttgttgc gcttgaggta ctcaaacagc gtgatgtcat catccggtcc 2660
   caggatatct tccaggaagt gacgcaactg accaggcttt tgcgggaagt tcaccaagaa 2720
   gtagtgcttc aaaccgcggt gcaccaagga gcgctcagcg atttccgcat aacgcagcac 2780
   atcgttgttg ccaccagaga tgatgcacac cacgacagaa ccaggtgcaa aggacatttc 2840
   cttcaaccca gcgatagaca gcgcgccagc aggctccgcg atgatgcctt cgttttggta 2900
   aagatcgagc atctcagtac acacagcgcc ctcggtcgcg ctcatcatgt gcacgcgacc 2960
   ctggttcttc tccacgatgg tgtagttgag atctccgaca cgtttgactg ctgcgccgtc 3020
   cacaaaggga tca                                                    3033
<210> 6
   <211> 610
   <212> PRT
   <213> Corynebacterium glutamicum
<400> 6
   Met Leu Lys Asp Leu Thr Gly Leu Arg Glu Leu Val Leu Arg Glu Met
   1               5                   10                  15
   Cys His Ser Ile Ser His Leu Ser Ser Pro Thr Gly Ser Ile Phe Thr
               20                  25                  30
   Ser Leu Val Ala Met Leu Thr Ser Gln Ser Phe Ser Val Trp Ala Pro
           35                  40                  45
   Leu Pro His Asp Val His Leu Ile Leu Asn Gly Glu Thr Leu Pro Met
       50                  55                  60
   His Lys Thr Glu Gly Ser Trp Trp Arg Ala Glu Ile Ala Pro Lys Ala
   65                  70                  75                  80
   Gly Asp Arg Tyr Gly Phe Ser Leu Phe Asp Gly Ser Ser Trp Ser Lys
                   85                  90                  95
   Thr Leu Pro Asp Pro Arg Ser Thr Ser Gln Pro Asp Gly Val His Gly
               100                 105                 110
   Leu Ser Glu Val Ser Asp Asp Ser Tyr Leu Trp Gly Asp Gln Gln Trp
           115                 120                 125
   Thr Gly Arg Ile Leu Pro Gly Ser Val Leu Tyr Glu Leu His Val Gly
       130                 135                 140
   Thr Phe Ser Glu Asp Gly Thr Phe Glu Gly Val Val Asp Lys Leu Pro
   145                 150                 155                 160
   Tyr Leu Arg Asp Leu Gly Val Thr Ala Ile Glu Leu Leu Pro Val Gln
                   165                 170                 175
   Pro Phe Gly Gly Asn Arg Asn Trp Gly Tyr Asp Gly Val Leu Trp His
               180                 185                 190
   Ala Val His Ala Gly Tyr Gly Gly Pro Ala Gly Leu Lys Lys Leu Ile
           195                 200                 205
   Asp Ala Ser His Gln Ala Gly Ile Ala Val Tyr Leu Asp Val Val Tyr
       210                 215                 220
   Asn His Phe Gly Pro Asp Gly Asn Tyr Asn Gly Gln Phe Gly Pro Tyr
   225                 230                 235                 240
   Thr Ser Gly Gly Ser Thr Gly Trp Gly Asp Val Val Asn Ile Asn Gly
                   245                 250                 255
   His Asp Ser Asp Glu Val Arg Asn Tyr Ile Leu Asp Ala Ala Arg Gln
               260                 265                 270
   Trp Phe Glu Asp Phe His Val Asp Gly Leu Arg Leu Asp Ala Val His
           275                 280                 285
   Ser Leu Asp Asp Arg Gly Ala Tyr Ser Leu Leu Ala Gln Leu Thr Met
   290 295 300
   Val Ala Glu Asp Val Ser Ala Gln Thr Gly Ile Pro Arg Ser Leu Ile
   305 310 315 320
   Ala Glu Ser Glu Leu Asn Asp Pro Lys Phe Val Thr Ser Arg Glu Ala
   325 330 335
   Gly Gly Phe Gly Leu Asp Ala Gln Trp Val Asp Asp Ile His His Ala
   340 345 350
   Leu His Ala Leu Val Ser Gly Glu Arg Asn Gly Tyr Tyr Ser Asp Phe
   355 360 365
   Gly Ser Val Asp Thr Leu Ala Lys Thr Leu Arg Glu Val Phe Glu His
   370 375 380
   Thr Gly Asn Tyr Ser Thr Tyr Arg Gly Arg Asn His Gly Arg Pro Val
   385 390 395 400
   His Pro Asp Ile Thr Pro Ala Ser Arg Phe Val Thr Tyr Thr Thr Thr
   405 410 415
   His Asp Gln Thr Gly Asn Arg Ala Ile Gly Asp Arg Pro Ser Thr Thr
   420 425 430
   Leu Thr Pro Glu Gln Gln Val Leu Lys Ala Ala Ile Ile Tyr Ser Ser
   435 440 445
   Pro Tyr Thr Pro Met Leu Phe Met Gly Glu Glu Phe Gly Ala Thr Thr
   450 455 460
   Pro Phe Ala Phe Phe Cys Ser His Thr Asp Pro Glu Leu Asn Arg Leu
   465 470 475 480
   Thr Ser Glu Gly Arg Lys Arg Glu Phe Ala Arg Leu Gly Trp Asn Ala
   485 490 495
   Asp Asp Ile Pro Ser Pro Glu Leu Glu Ser Thr Phe Thr Ser Ser Lys
   500 505 510
   Leu Asp Trp Glu Phe Thr Ala Glu Gln Arg Arg Ile Asn Asp Ala Tyr
   515 520 525
   Lys Gln Leu Leu His Leu Arg His Thr Leu Gly Phe Ser Gln Pro Asn
   530 535 540
   Leu Leu Thr Leu Glu Val Glu His Gly Glu Asn Trp Leu Ser Met Ala
   545 550 555 560
   Asn Gly Arg Gly Arg Ile Leu Ala Asn Phe Ser Asp Asp Thr Ile Thr
   565 570 575
   Val Pro Leu Gly Gly Glu Leu Ile Tyr Ser Phe Thr Ser Pro Thr Val
   580 585 590
   Thr Asp Thr Ser Thr Thr Leu Gln Pro Trp Gly Phe Ala Ile Leu Thr
   595 600 605
   Arg Asn
       610
<210> 7
   <211> 19
   <212> DNA
   <213> Corynebacterium glutamicum
<220>
   <223> Primer otsB-int1
<400> 7
   gtccgatttt gatggaacc 19
<210> 8
   <211> 19
   <212> DNA
   <213> Corynebacterium glutamicum
<220>
   <223> Primer otsB-int2
<400> 8
   ggagctgatg gagtattcg 19
<210> 9
   <211> 19
   <212> DNA
   <213> Corynebacterium glutamicum
<220>
   <223> Primer treY-int1
<400> 9
   ttttccgtga atacgttgg 19
<210> 10
   <211> 19
   <212> DNA
   <213> Corynebacterium glutamicum
<220>
   <223> Primer treY-int2
<400> 10
   gcgactaatt cgatgatgg 19
<210> 11
   <211> 19
   <212> DNA
   <213> Corynebacterium glutamicum
<220>
   <223> Primer treZ-int1
<400> 11
   tggttcgaag attttcacg 19
<210> 12
   <211> 19
   <212> DNA
   <213> Corynebacterium glutamicum
<220>
   <223> Primer treZ-int2
<400> 12
   ggcgagctgt agataatgg
   19